# EUROPEAN PATENT APPLICATION

(11) **EP 3 392 709 A1**
(43) Date of publication of application: **24.10.2018**
(21) Application number: 17167515.0
(22) Date of filing: 21.04.2017
(51) Int. Cl.: G03F 7/031, C07D 233/64

(54) **A LITHOGRAPHIC PRINTING PLATE PRECURSOR**

(71) Applicant: AGFA NV, 2640 Mortsel (BE)
(72) Inventor: Hendrikx, Peter, 2640 Mortsel (BE); Loccufier, Johan, 2640 Mortsel (BE); Verbrugghe, Sam, 2640 Mortsel (BE)
(74) Representative: Vanderstede, Els

(57) **Abstract**

A negative-working lithographic printing plate precursor is disclosed including (i) a support having a hydrophilic surface or which is provided with a hydrophilic layer, and (ii) a coating including a photopolymerisable layer including a photoinitiator and a polymerizable compound, characterised in that the photoinitiator includes at least one structural moiety including an oligo- or poly(alkyleneglycol).

## Description

### FIELD OF THE INVENTION

The present invention relates to a negative working lithographic printing plate precursor comprising in its coating a photoinitiator including a structural moiety including an oligo or (poly)alkyleneoxide.

### BACKGROUND OF THE INVENTION

Lithographic printing presses use a so-called printing master such as a printing plate which is mounted on a cylinder of the printing press. The master carries a lithographic image on its surface and a print is obtained by applying ink to said image and then transferring the ink from the master onto a receiver material, which is typically paper. In conventional, so-called "wet" lithographic printing, ink as well as an aqueous fountain solution (also called dampening liquid) are supplied to the lithographic image which consists of oleophilic (or hydrophobic, i.e. ink-accepting, water-repelling) areas as well as hydrophilic (or oleophobic, i.e. water-accepting, ink-repelling) areas. In so-called driographic printing, the lithographic image consists of ink-accepting and ink-abhesive (ink-repelling) areas and during driographic printing, only ink is supplied to the master.

The so-called "analogue" printing plates are generally obtained by first applying a so-called computer-to-film (CtF) method, wherein various pre-press steps such as typeface selection, scanning, color separation, screening, trapping, layout and imposition are accomplished digitally and each color selection is transferred to graphic arts film using an imagesetter. After processing, the film can be used as a mask for the exposure of an imaging material called plate precursor and after plate processing, a printing plate is obtained which can be used as a master. Since about 1995, the so-called "computer-to-plate" (CtP) method has gained a lot of interest. This method, also called "direct-to-plate", bypasses the creation of film because the digital document is transferred directly to a printing plate precursor by means of a platesetter. A printing plate precursor for CtP is often called a digital plate.

The support of the lithographic printing plates are typically aluminum supports which have a hydrophilic surface or on which a hydrophilic layer has been provided. This hydrophilic surface and/or layer should improve the water acceptance of the non-printing areas of a lithographic printing plate and the repulsion of the printing ink in these areas. During developing the soluble portions of the coating should be easily removed whereby the surface of the support remains residue-free so that clean background areas are obtained during printing.

Digital plates can roughly be divided in three categories: (i) silver plates, working according to the silver salt diffusion transfer mechanism; (ii) photopolymer plates containing a photopolymerisable composition that hardens upon exposure to light and (iii) thermal plates of which the imaging mechanism is triggered by heat or by light-to-heat conversion.

Photopolymer printing plates rely on a working-mechanism whereby the coating - which typically includes free radically polymerisable compounds - hardens upon exposure. "Hardens" means that the coating becomes insoluble or non-dispersible in the developing solution and may be achieved through polymerization and/or crosslinking of the photosensitive coating upon exposure to light. Photopolymer plate precursors can be sensitized to blue, green or red light i.e. wavelengths ranging between 450 and 750 nm, to violet light i.e. wavelengths ranging between 350 and 450 nm or to infrared light i.e. wavelengths ranging between 750 and 1500 nm. Optionally, the exposure step is followed by a heating step to enhance or to speed-up the polymerization and/or crosslinking reaction. The presslife of photopolymer plates is related to the cohesive strength within the polymerized photolayer. The higher the cohesive strength, the higher the presslife. The cohesive strength can preferably be improved by increasing the crosslinking degree and/or by supramolecular non-covalent interactions such as H-bonding, Van der Waals interaction and dipole-dipole interactions.

In general, a toplayer or protective overcoat layer over the imageable layer is required to act as an oxygen barrier to provide the desired sensitivity to the plate. A toplayer typically includes water-soluble or water-swellable polymers such as for example polyvinylalcohol.

Photopolymer plates generally contain a polymerizable monomer, a binder, a photo-initiator and a sensitizing dye. Well known and widely used photo-initiators are hexa-arylbisimidazole compounds, i.e. HABI-compounds, for example disclosed in EP 1 757 981, WO2008/145528 (page 14 lines 1 to 26), EP 1 757 981 ([0054] and [0055]), EP 1 748 317 ([0046] and [0047]) and WO2007/090550 (page 25-27), EP 24 629, EP 107 792, US 4,410,621, US 8,034,538 (column 6 and column 7), EP 215 453 and DE 3 211 312.

The classical workflow for making photopolymer plates involves first an exposure step of the photopolymer printing plate precursor in a violet or infrared platesetter, followed by an optional pre-heat step, a wash step of the protective overcoat layer, an alkaline developing step, and a rinse and gum step. Over the past years, there is a clear evolution in the direction of a simplified workflow where the pre-heat step and/or wash step are eliminated and where the development and gumming step are carried out in one single step. Indeed, in the art, there is a constant demand to reduce waste, simplify the processing step and to reduce the amount of processing liquid needed per square meter.

A major problem associated with processing of printing plate precursors is that non-image areas which are dissolved into the developer during processing may - possibly together with other components of the developer - precipitate, flocculate, salt-out (i.e. organic sludge) or gelatinize (i.e. formation of gelatinous and/or turbid areas) making the maintenance of the processing bath more burdensome. In addition, these non-image areas may deposit on the exit rollers of the developer section, build-up on the heater elements in the developer section and/or clogg pumps. Indeed, it has been observed in the art that upon prolonged processing of violet sensitized printing plate precursors, yellow stain is often formed in the clean out unit caused by deposition of such different plate components leading to difficult and laborious cleaning procedures and the need for cleaning liquids containing aggressive solvents. In the current invention this deposition of different components is also referred to as "deposits", which are often yellow. This not only induces productivity loss due to down time of the plate making line, but also leads to generation of hazardous waste and health and safety risks.

Therefore, there is a clear demand in the market for a more ecological type of processing, with a high preference for single step processing that allow high exhaustion levels and/or whereby formation of sludge is highly reduced and the need for laborious cleaning of the clean out units is avoided.

### SUMMARY OF THE INVENTION

It is an object of the present invention to provide a negative-working lithographic printing plate precursor including a photopolymerisable layer which does not generate substantive deposits during processing.

This object is realized by the printing plate precursor defined in claim 1, i.e. a negative-working lithographic printing plate precursor including a support having a hydrophilic surface or which is provided with a hydrophilic layer, and a coating including a photopolymerisable layer including a compound comprising at least one free radically polymerisable group and a photoinitiator including at least one structural moiety including oligo- or poly(alkylene glycol).

It is a further object of the present invention to provide a method for making a lithographic printing plate whereby during the processing step the formation of deposits, i.e. organic sludge, precipitates and/or deposit materials, in the developer solution are minimised or even avoided.

It was surprisingly found that the solubility of the components present in a developer solution during and/or after processing of printing plate precursors containing a coating including the novel photoinitiator, is significantly improved compared to the solubility during and/or after processing of printing plate precursors including photoinitiators of the prior art. With an improved solubility of the components present in a developer solution is meant that the tendency of the developer to precipitate, flocculate, salt-out or gelatinize is reduced.

Other features, elements, steps, characteristics and advantages of the present invention will become more apparent from the following detailed description of preferred embodiments of the present invention. Specific embodiments of the invention are also defined in the dependent claims.

### DETAILED DESCRIPTION OF THE INVENTION

The lithographic printing plate precursor according to the present invention is negative-working, i.e. after exposure and development the non-exposed areas of the coating are removed from the support and define hydrophilic (non-printing) areas, whereas the exposed coating is not removed from the support and defines oleophilic (printing) areas. The hydrophilic areas are defined by the support which has a hydrophilic surface or is provided with a hydrophilic layer. The hydrophobic areas are defined by the coating, hardened upon exposing, optionally followed by a heating step. Areas having hydrophilic properties means areas having a higher affinity for an aqueous solution than for an oleophilic ink; areas having hydrophobic properties means areas having a higher affinity for an oleophilic ink than for an aqueous solution.

"Hardened" means that the coating becomes insoluble or non-dispersible for the developing solution and may be achieved through polymerization and/or crosslinking of the photosensitive coating, optionally followed by a heating step to enhance or to speed-up the polymerization and/or crosslinking reaction. In this optionally heating step, hereinafter also referred to as "pre-heat", the plate precursor is heated, preferably at a temperature of about 80°C to 150°C and preferably during a dwell time of about 5 seconds to 1 minute.

### Photoinitiator

The coating of the printing plate precursor includes a photoinitiator including at least one structural moiety including oligo- or poly(alkylene glycol); also referred to herein as "the photoinitiator". Preferably, the photoinitiator is a substituted bisimidazole based photoinitiator including at least one structural moiety including oligo- or poly(alkylene glycol). Oligo (alkylene glycol) refers to a structure including a limited amount of monomers such as two, three or four repeating units; and poly (alkylene glycol) refers to a structure including more than four repeating units. The structural moiety including oligo or poly(alkylene glycol) is preferably represented by Formula I:

*-(L)ₚ-(OCHR^{a}CH₂)ₙ-(OCHR^{b}CH₂)ₘ-R Formula I

wherein
n represents an integer from 2 to 50;
m represents an integer from 0 to 50;
p represents 0 or 1;
L represents a linking group;
R represents a terminal group;
R^{a} and R^{b} independently represents hydrogen, an optionally substituted alkyl group and/or mixtures thereof; preferably R^{a} # R^{b} and
* denotes the linking position to the remaining portion of the photoiniator.

Preferably, n is an integer comprised between 2 and 35, more preferably between 3 and 20 and most preferably between 5 and 15. Preferably, m is an integer comprised between 2 and 35, more preferably between 3 and 20 and most preferably between 5 and 15. In a highly preferred embodiment, m is 0 and n is an integer comprised between 2 and 35.

Suitable alkyl groups are described below. In a further preferred embodiment, R^{a} and R^{b} independently represent hydrogen, a methyl group or hydroxymethyl group. More preferably R^{a} represents hydrogen and R^{b} represents methyl or hydroxymethyl.

Most preferably, m is 0 and R^{a} is hydrogen and the structural moiety including oligo or poly(alkylene glycol) is preferably poly(ethylene glycol) represented by Formula II:

*-(L)ₚ-(OCH₂CH₂)ₙ-R Formula II

wherein
n, p, L, R and * represent the same as defined for Formula I.

The linking group L is preferably divalent and independently selected from an optionally substituted alkylene or cycloalkylene group, an optionally substituted arylene or heteroarylene, -O-, -CO-, -CO-O-, -O-CO-, -CO-NH-, -NH-CO-, -NH-CO-O-, -O-CO-NH-, -NH-CO-NH-, -NH-CS-NH-, -CO-NR'-, -NR"-CO-, - SO-, -SO₂-, -SO₂-NH-, -NH-SO₂-, -CH=N-, -NH-NH-, -S-, -S-S-, -NH-CO-CO-NH-, and/or combinations thereof, wherein R' and R" each independently represent an optionally substituted alkyl, aryl, aralkyl or heteroaryl group.

Preferably, the linking group L represents a divalent aliphatic group including straight or branched carbon chain(s) or alicyclic, non-aromatic ring(s). Optionally the aliphatic linking group may contain substituents including for example oxygen or sulfur; alkyl groups such as a methyl, ethyl, propyl or isopropyl group and halogens such as a fluoro, chloro, bromo or iodo atom.

The terminal group R may represent hydrogen, hydroxy, an alkoxy such as methoxy or ethoxy, or an optionally substituted alkyl, aryl, aralkyl or heteroaryl group. Most preferably the terminal group represents a C₁ to C₆-alkoxy group.

Preferably, the photoinitiator is represented by Formula III: 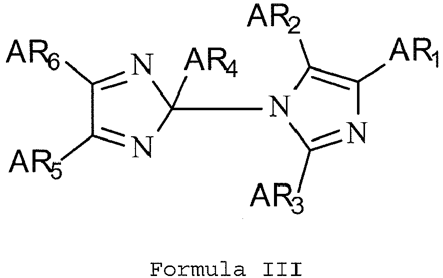 wherein
AR₁ to AR₆ independently represent an optionally substituted aryl, aralkyl or heteroaryl group;
with the proviso that at least one of AR₁ to AR₆ includes at least one structural moiety including oligo- or poly(alkylene glycol).

Preferably, at least two of AR₁ to AR₆ include at least one structural moiety including oligo- or poly(alkylene glycol); more preferably at least three of AR₁ to AR₆ include at least one structural moiety including oligo- or poly(alkylene glycol); and most preferably at least four or more of AR₁ to AR₆ include at least one structural moiety including oligo- or poly(alkylene glycol). The oligo- or poly(alkylene glycol) is preferably as defined above (Formula I and Formula II).

One or more of AR₁ to AR₆ may be substituted with two or more structural moieties including oligo- or poly(alkylene glycol).

A suitable optionally substituted aryl group is selected from an optionally substituted phenyl, naphthyl, tolyl, ortho-meta- or para-xylyl, anthracenyl or phenanthrenyl. Preferably, the optionally substituted aryl group is selected from an optionally substituted phenyl, naphthyl or tolyl group. Most preferably, the optionally substituted aryl group is selected from an optionally substituted phenyl or naphthyl group.

A suitable aralkyl group includes for example a phenyl group or a naphthyl group including one, two, three or more C₁ to C₆-alkyl groups.

A suitable heteroaryl group is preferably a monocyclic- or polycyclic aromatic ring - preferably a five or six membered ring - comprising carbon atoms and one or more heteroatoms in the ring structure. Preferably 1 to 4 heteroatoms are independently selected from nitrogen, oxygen, selenium and sulphur and/or combinations thereof. Examples include pyridyl, pyrimidyl, pyrazoyl, triazinyl, imidazolyl, (1,2,3,)- and (1,2,4)-triazolyl, tetrazolyl, furyl, thienyl, isoxazolyl, thiazolyl and carbazoyl.

The term "substituted", in e.g. substituted alkyl group means that the alkyl group may be substituted by other atoms than the atoms normally present in such a group, i.e. carbon and hydrogen. An unsubstituted alkyl group contains only carbon and hydrogen atoms. For example, a substituted alkyl group may include an ester, amine, (di)alkylamine, amide, ether, thioether, ketone, aldehyde, thiol, sulfoxide, sulfone, sulfonate ester or sulphonamide group, a halogen such as a fluoro, chloro, bromo or iodo, a hydroxyl group, -SH, -CN and -NO₂, or an alkoxy group such as a methoxy or ethoxy group. A halogen and a hydroxyl group are preferred.

Preferred substituents optionally present on the other groups defined above include an alkyl such as a methyl, ethyl, n-propyl, isopropyl, n-butyl, isobutyl and tertiary-butyl, an ester, amine, (di)alkylamine, amide, nitro, cyanide, ether, thioether, ketone, aldehyde, thiol, sulfoxide, sulfone, sulfonate ester or sulphonamide group, a halogen such as a fluoro, chloro, bromo or iodo, a hydroxyl group, -SH, -CN and -NO₂, or an alkoxy group such as a methoxy or ethoxy group. More preferably, the optional substituents are represented by a halogen such as a fluoro, chloro, bromo or iodo atom, a hydroxyl group, an amine group, a (di)alkylamine group or an alkoxy group such as a methoxy or ethoxy group.

Preferably, the photoinitiator is a hexaarylbisimidazool and AR₁ to AR₆ independently represent an optionally substituted aryl group as defined above. Most preferably, the optionally substituted aryl group is an optionally substituted phenyl or naphthyl group.

Most preferably, the photoinitiator has a structure according to Formula IV: 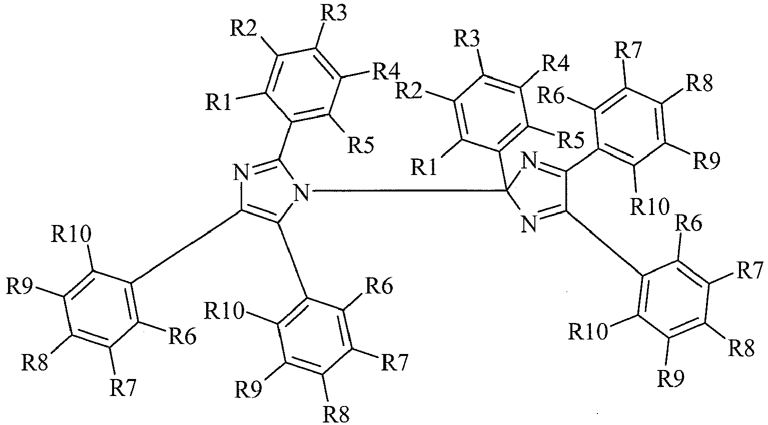 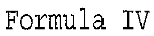 wherein
R¹ to R¹⁰ are independently selected from hydrogen, an alkyl group, an alkenyl group, an alkynyl group, an aralkyl group an alkaryl group, a (hetero)aryl group, an alkoxy group, a hydroxyl group, a (hetero)aryloxy group, a halogen, a carboxyl group, an ester, an amide, a nitro group and a nitrile group; with the proviso that at least one of R¹ to R¹⁰ represents the structural moiety including oligo- or poly(alkylene glycol) as described above.

Preferably, R¹ to R¹⁰ are independently selected from hydrogen, an alkyl group, a halogen or an alkoxy group, with the proviso that at least one of R⁶ to R¹⁰ represents the structural moiety including oligo- or poly(alkylene glycol) as described above.

More preferably, R¹ represents a halogen such as a fluoro, chloro, bromo or iodo atom, preferably a chloro atom, and R² to R¹⁰ represent hydrogen with the proviso that at least one of R⁶ to R¹⁰ represents the structural moiety including oligo- or poly(alkylene glycol) as described above; more preferably at least two of R⁶ to R¹⁰ represents the structural moiety including oligo- or poly(alkylene glycol) as described above; most preferably at least four of R⁶ to R¹⁰ represents the structural moiety including oligo- or poly(alkylene glycol) as described above.

In the present invention, a suitable alkenyl or alkynyl group is respectively a C2 to C₆-alkenyl group or a C₂ to C₆-alkynyl group. Suitable aralkyl, aryl or heteroaryl groups are as discussed above. An alkaryl group is preferably a C₇ to C₂₀-alkyl group including a phenyl group or naphthyl group.

In the present invention, suitable alkyl groups include 1 or more carbon atoms such as for example C₁ to C₂₂-alkyl groups, more preferably C₁ to C₁₂-alkyl groups and most preferably C₁ to C₆-alkyl groups. The alkyl group may be lineair or branched such as for example methyl, ethyl, propyl (n-propyl, isopropyl), butyl (n-butyl, isobutyl, t-butyl), pentyl, 1,1-dimethyl-propyl, 2,2-dimethylpropyl and 2-methyl-butyl, or hexyl. The alkyl group may be cyclic; suitable cycloalkyl groups are non-aromatic, homocyclic groups containing carbon atoms and may be monocyclic- or polycyclic. Examples include cyclopentyl, cyclohexyl or adamantyl.

Without being limited thereto, typical examples of the photoinitiator of the present invention are given below.

| Initiator | Chemical structure |
|---|---|
| PEG-HABI 1 | 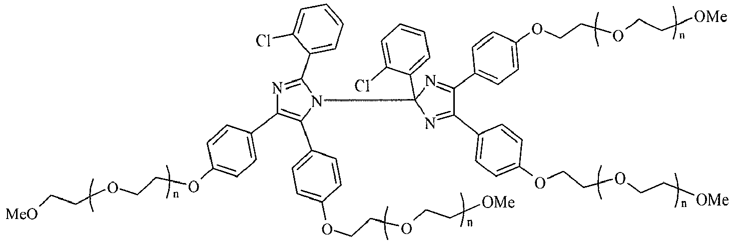 |
| n = 16 on average | |
| PEG-HABI 2 | 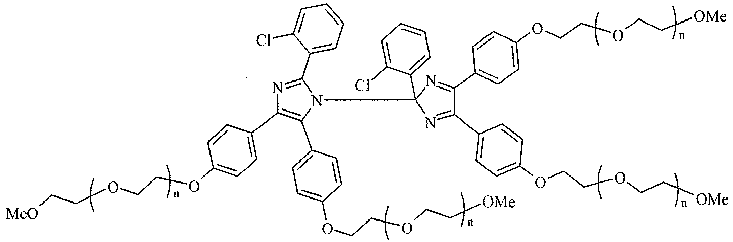 |
| n = 7 on average | |
| PEG-HABI 3 | 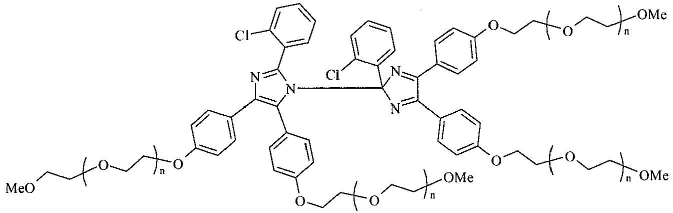 |
| n = 12 on average | |
| PEG-HABI 4 | 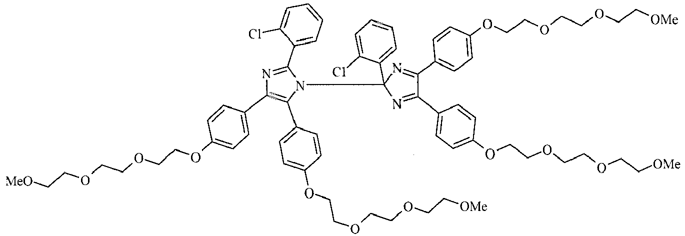 |
| PEG-HABI 5 | 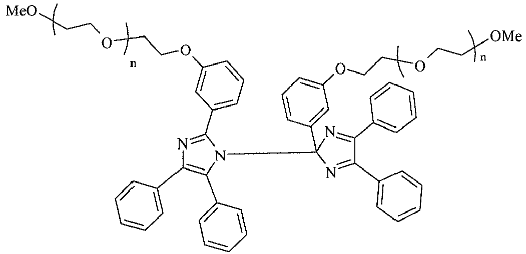 |
| n = 7 on average | |
| PEG-HABI 6 | 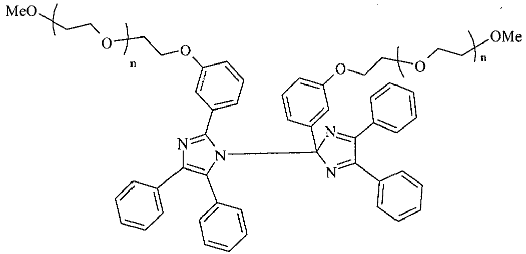 |
| n = 12 on average | |
| PEG-HABI 7 | 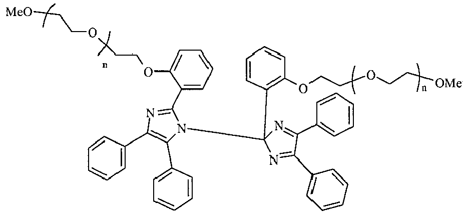 |
| n = 3 on average | |
| PEG-HABI 8 | 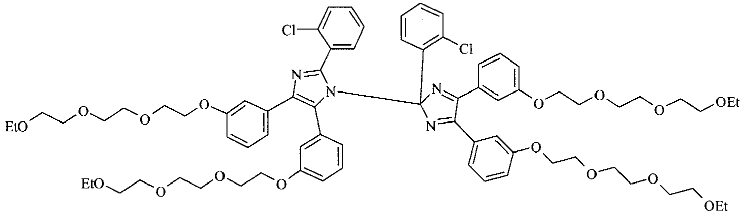 |
| PEG-HABI 9 | 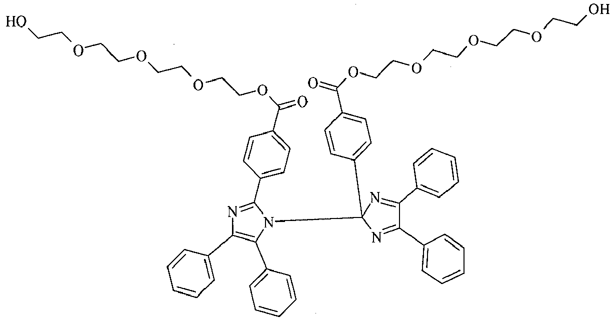 |
| PEG-HABI 10 | 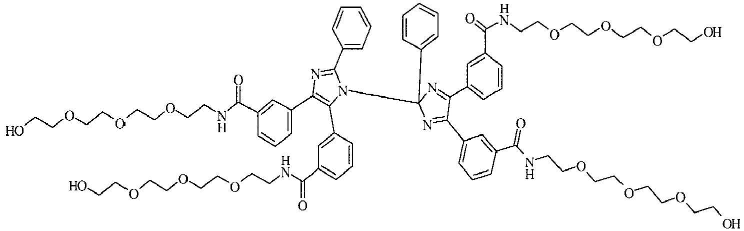 |
| PEG-HABI 11 | 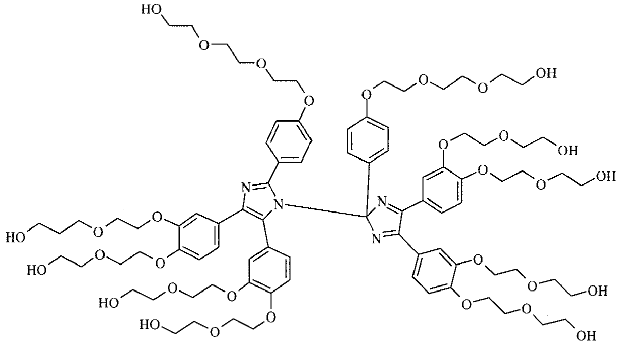 |
| PEG-HABI 12 | 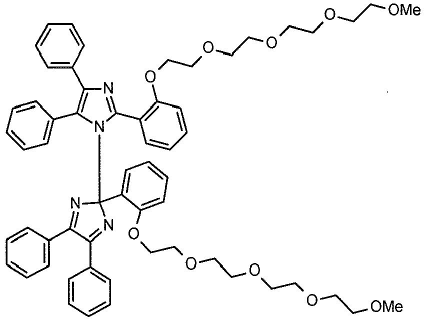 |
| PEG-HABI 13 | 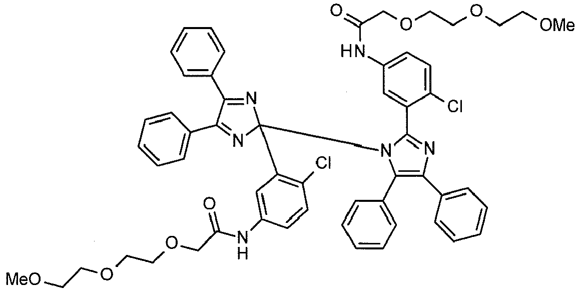 |
| PEG-HABI 14 n = 7 on average | 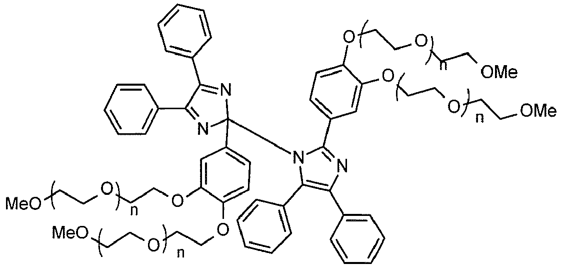 |
| PEG-HABI 15 | 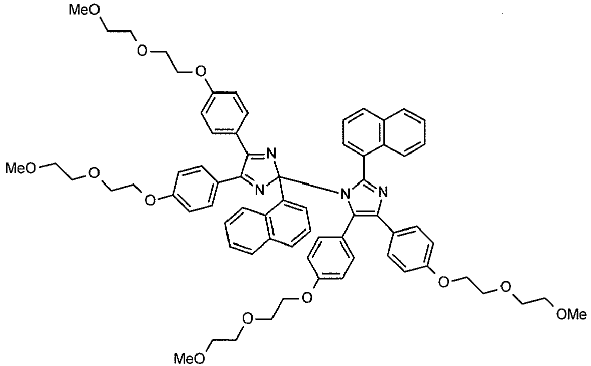 |
| PEG-HABI 16 n=7 on average | 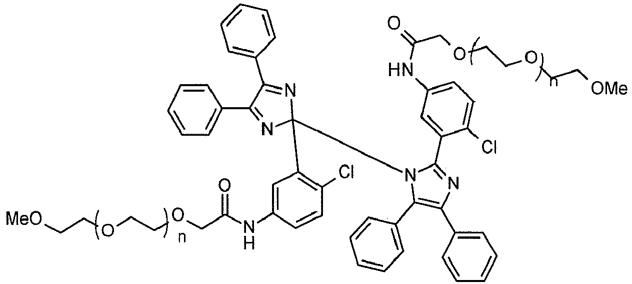 |
| PEG-HABI 17 n=12 on average | 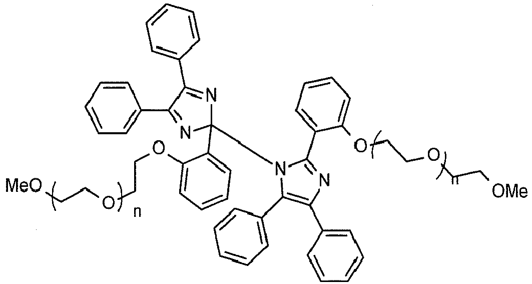 |
| PEG-HABI 18 | 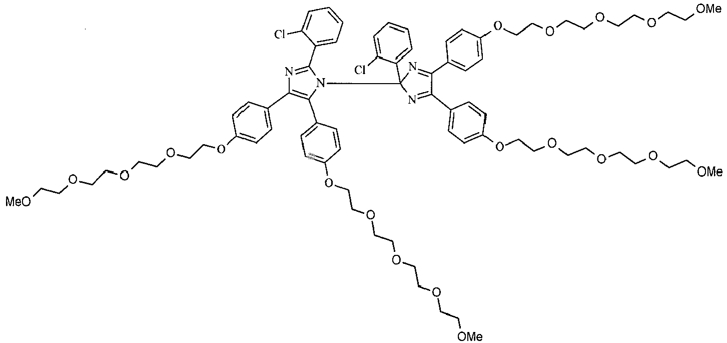 |

### The coating, photoinitiator

The coating has at least one layer including the photopolymerisable composition, said layer is also referred to as the "photopolymerisable layer". The coating may include an intermediate layer, located between the support and the photopolymerisable layer.

The photopolymerisable layer includes the photoinitiator of the present invention capable of hardening a polymerisable compound in the exposed areas, preferably in an amount of above 1 %wt, more preferably above 2 %wt and most preferably above 5% wt relative to the total weight of all ingredients in the photopolymerisable layer. Alternatively, the photoinitiator in the photopolymerisable layer is preferably between 0.1 and 30 %wt, more preferably between 1 and 20 %wt, most preferably between 5 and 15 %wt relative to the total weight of the non volatile components of the photopolymerisable layer.

The photopolymerisable composition may comprise beside the photoinitiator of the present invention, other polymerisation initiators such as for example 2,4,5,2',4',5'-hexaphenylbisimidazole, 2,2'-bis(2-chlorophenyl)-4,5,4',5'-tetraphenylbisimidazole, 2,2'-bis(2-chlorophenyl)-4,5,4',5'-tetrakis(3-methoxyphenyl)bisimidazole and 2,2'-bis(2-nitrophenyl)-4,5,4',5'-tetraphenylbisimidazole. Further suitable classes of polymerisation initiators other than hexaarylbisimidazole compounds include aromatic ketones, aromatic onium salts, organic peroxides, thio compounds, ketooxime ester compounds, borate compounds, azinium compounds, metallocene compounds, active ester compounds and compounds having a carbon-halogen bond, but preferably the composition comprises a non-boron comprising polymerisation initiator and particularly preferred the polymerisation initiator comprises no boron compound. Many specific examples of initiators suitable for the present invention can be found in EP 1 091 247. Other preferred polymerization initiators are trihalo methyl sulphones. Suitable free-radical initiators are described in WO 2005/111727 from page 15 line 17 to page 16 line 11. Suitable cationic photoinitiators include, for example, triarylsulfonium hexafluoroantimonate, triarylsulfonium hexafluorophosphate, diaryliodonium hexafluoroantimonate, and haloalkyl substituted s-triazine. It is noted that most cationic initiators are also free radical initiators because, in addition to generating Brönsted acid, they also generate free radicals during photo or thermal decomposition.

### Co-initiators

Co-initiators, as described in EP 107 792, may be present in the photopolymerizable layer to further increase the sensitivity. Suitable co-initiators are disclosed in US 6,410,205; US 5,049,479; EP 1 079 276, EP 1 369 232, EP 1 369 231, EP 1 341 040, US 2003/0124460, EP 1 241 002, EP 1 288 720 and in the reference book including the cited references: Chemistry & Technology UV & EB formulation for coatings, inks & paints - Volume 3 -Photoinitiators for Free Radical and Cationic Polymerisation by K.K. Dietliker - Edited by P.K.T. Oldring -1991-ISBN 0 947798161. Specific co-initiators, as described in EP 107 792, may be present in the photopolymerizable layer to further increase the sensitivity. Preferred co-initiators are sulfur-compounds, especially thiols like e.g. 2-mercaptobenzothiazole, 2-mercaptobenzoxazole, 2-mercapto-benzimidazole, 4-methyl-3-propyl-1,2,4-triazoline-5-thione, 4-methyl-3-n-heptyl-1,2,4-triazoline-5-thione, 4-phenyl-3-n-heptyl-1,2,4-triazoline-5-thione,4-phenyl-3,5-dimercapto-1,2,4-triazole, 4-n-decyl-3,5-dimercapto-1,2,4-triazole, 5-phenyl-2-mercapto-1,3,4-oxadiazole, 5-methylthio-1,3,4-thiadiazoline-2-thione, 5-hexylthio-1,3,4-thiadiazoline-2-thione, mercaptophenyltetrazole, pentaerythritol mercaptopropionate, butyric acid-3-mercapto-neopentanetetrayl ester, pentaerythritol tetra(thioglycolate). Other preferred co-initiators, also referred to as chain-transfer agents are disclosed in EP 1 748 317, paragraphs [0091] to [0098]. Polythioles as disclosed in WO2006/048443 and WO2006/048445 may also be used.

The amount of co-initiator preferably ranges from 0.01 to 20 wt.%, more preferably from 0.1 to 15 wt.%, most preferably from 0.25 to 10 wt.% relative to the total weight of the non volatile components of the photopolymerizable composition.

### Sensitizers

The photopolymerisable layer may further include a sensitizer capable of absorbing light used in the image-wise exposing step. The photopolymer printing plate precursors may be sensitive to ultraviolet light, visible light and/or infrared light. Preferably, they are sensitized for (ultra)violet light, i.e. for light having a wavelength ranging from 350 nm to 500 nm, or for infrared light, i.e. light having a wavelength ranging from 750 nm to 1500 nm.

A sensitizer is a compound capable of transferring its excitation energy to a receptor molecule, for example the photoinitiator. For violet sensitive printing plate precursors, the sensitizer typically absorbs light having a wavelength between 350 and 500 nm, preferably between 375 and 450 nm, more preferably between 390 nm and 415 nm.

Any violet sensitizer may be used in the present invention but preferred sensitizers are those disclosed in for example EP 1 748 317 (paragraph [0016] to [0044] and EP 2 028 548 (paragraph [41] to [46]). Particularly preferred sensitizers are those disclosed in WO2005/029187, WO2008/145528 (page 5, ln.11 to page 13, ln.12), WO2008/145529 (page 5, ln.14 to page 13, ln.21), WO2008/145530, EP 1 349 006 paragraph [0007] to [0009], EP 1 668 417 and WO 2004/047930, including the cited references in these patent applications.

Suitable examples of optical brighteners as sensitizers are described in WO 2005/109103 page 24, line 20 to page 39.

### Polymerisable compound

The photopolymerisable layer further includes a polymerisable compound and optionally a binder. The photopolymerisable layer has a coating thickness preferably ranging between 0.2 and 5.0 g/m², more preferably between 0.4 and 3.0 g/m², most preferably between 0.6 and 2.2 g/m².

According to a preferred embodiment, the polymerisable compound is a monomer or oligomer including at least one epoxy or vinyl ether functional group and the polymerisation initiator is a Brönsted acid generator capable of generating free acid, optionally in the presence of a sensitizer, upon exposure, hereinafter the Brönsted acid generator is also referred to as "cationic photoinitiator" or "cationic initiator".

Suitable polyfunctional epoxy monomers include, for example, 3,4-epoxycyclohexylmethyl-3,4-epoxycyclohexane carboxylate, bis-(3,4 -epoxycyclohexymethyl) adipate, difunctional bisphenol A-epichlorohydrin epoxy resin and multifunctional epichlorohydrintetraphenylol ethane epoxy resin.

According to a more preferred embodiment of the present invention, the polymerisable compound is a polymerisable monomer or oligomer including at least one terminal ethylenic group, hereinafter also referred to as "free-radical polymerisable monomer", and the polymerisation initiator is a compound capable of generating free radicals upon exposure, optionally in the presence of a sensitizer, hereinafter said initiator is referred to as "free radical initiator". The polymerisation involves the linking together of the free-radical polymerisable monomers.

Suitable free-radical polymerisable monomers include, for example, multifunctional (meth)acrylate monomers (such as (meth)acrylate esters of ethylene glycol, trimethylolpropane, pentaerythritol, ethoxylated ethylene glycol and ethoxylated trimethylolpropane, multifunctional urethanated (meth)acrylate, and epoxylated (meth)acrylate), and oligomeric amine diacrylates. The (meth)acrylic monomers may also have other double bond or epoxide groups, in addition to (meth)acrylate group. The (meth)acrylate monomers may also contain an acidic (such as carboxylic acid) or basic (such as amine) functionality.

According to another embodiment of the present invention, the polymerisable monomer or oligomer may be a combination of a monomer or oligomer comprising at least one epoxy or vinyl ether functional group and a polymerisable ethylenically unsaturated compound having at least one terminal ethylenic group. A monomer or oligomer comprising at least one epoxy or vinyl ether functional group and a polymerisable ethylenically unsaturated compound having at least one terminal ethylenic group, can be the same compound wherein the compound contains both the ethylenic group and the epoxy or vinyl ether group. Examples of such compounds include epoxy functional acrylic monomers, such as glycidyl acrylate.

The photopolymerisable layer may also comprise a multifunctional monomer. This monomer contains at least two functional groups selected from an ethylenically unsaturated group and/or an epoxy or vinyl ether group. Particular multifunctional monomers for use in the photopolymer coating are disclosed in US 6,410,205 , US 5,049,479 , EP 1 079 276 , EP 1 369 232 , EP 1 369 231, EP 1 341 040 , US 2003/0124460 , EP 1 241 002 , EP 1 288 720 and in the reference book including the cited references: Chemistry & Technology UV & EB formulation for coatings, inks & paints - Volume 2 -Prepolymers and Reactive Diluents for UV and EB Curable Formulations by N.S. Allen, M.A. Johnson, P.K.T. Oldring, M.S. Salim - Edited by P.K.T. Oldring - 1991 - ISBN 0 947798102. Particularly preferred are urethane (meth)acrylate multifunctional monomers, which can be used alone or in combination with other (meth)acrylate multifunctional monomers.

### Binder

The photopolymerisable layer may include a binder. The binder can be selected from a wide series of organic polymers. Compositions of different binders can also be used. Useful binders are described in WO2005/052298 page 17 line 21 to page 19 line 30, in EP 152 819 on page 2 line 50 to page 4 line 20, and in EP 1 043 627 in paragraph [0013].

Preferred polymeric binders are polyvinyl acetate, partially hydrolyzed polyvinyl acetate, polyethylene oxide, copolymers of ethylene oxide and/or propylene oxide, polyurethane, polyurea, derivates of cellulose, gum Arabic, and copolymers containing at least one of the following monomeric units selected from vinyl acetate, vinyl alcohol, vinyl caprolactam, vinyl pyrrolidone, alkylated vinyl-pyrrolidone, alkyl (meth)acrylate, hydroxy-alkyl (meth)acrylate, (meth)acrylamide and monomeric units having a sulphonate group. Particularly preferred polymeric binders are partially hydrolyzed polyvinylacetate. Also preferred are binders with urethane and/or urea moieties in their backbones. For example, some useful first polymeric binders are polyurethanes that have NH-CO-O units or linkage in the polymer chain. They are generally obtained through the reaction of a diisocyanate with a diol. For example, aromatic or aliphatic diisocyanates can be used for this purpose. Other useful binders are polyureas that contain urea linkages, NH-CO-NH, formed from compounds bearing up to two amine groups that are reactive toward urethane groups.

The binder may be a mixture of polymeric compounds. It has been observed that good results are obtained, especially when the image-recording layer or an intermediate layer comprises adhesion promoting agents, when the image-recording layer comprises a polymer having at least 1 mol % of a first monomeric unit having a group capable of interacting with the support such as a phosphate group, a phosphonate group, a carboxylic acid group, a sulphonic acid group, a phenolic group, a trialkoxysilane group, an ammonium group or a phosphonium group and at least 30 mol % of a second monomeric unit having a hydrophilic group. Preferred embodiments of such a polymer are disclosed in WO2009/063024 on Page 16, ln.32 to page 19, ln.17. However, it is preferred that the amount of a polymer comprising monomeric units having -COOH, -PO₃H₂ or -OPO₃H₂ groups is less than 0.1 g/m².

The organic polymers used as binders have a typical average molecular weight M_{w} between 1000 and 700 000, preferably between 1500 and 350 000. Preferably, the binders have a hydroxyl number between 0 and 750, more preferably between 10 and 500. Even more preferably the hydroxyl number is below 10, most preferably the hydroxyl number is 0. The amount of binder(s) generally ranges from 1 to 60 % by weight, preferably 5 to 50 % by weight, more preferably 10 to 35 % by weight and most preferably 15 to 25 % by weight relative to the total weight of the non-volatile components of the composition.

In another embodiment the polymeric binder comprises a backbone including pendant groups such as for example a hydrophilic poly(alkylene oxide) segment. The polymeric binder may also include pendant cyano groups attached to the backbone. A combination of such binders may also be employed. Generally the polymeric binder is solid at room temperature, and is typically a non-elastomeric thermoplastic. Generally the polymeric binder is characterized by a number average molecular weight (Mn) in the range from about 500 to 250000, more commonly in the range from about 1000 to 240000 or 1500 to 200000. The polymerisable composition may comprise discrete particles of the polymeric binder. Preferably the discrete particles are particles of the polymeric binder which are suspended in the polymerisable composition. The presence of discrete particles tends to promote developability of the unexposed areas. Specific examples of the polymeric binders according to this embodiment are described in US 6,899,994; US 2004/0260050, US 2005/0003285, US 2005/0170286 and US 2005/0123853. In addition to the polymeric binder of this embodiment the imageable layer may optionally comprise one or more co-binders. Typical co-binders are water-soluble or water-dispersible polymers, such as, cellulose derivatives, polyvinylalcohol, polyacrylic acid, poly(meth)acrylic acid, polyvinylpyrrolidone, polylactide, polyvinylphosphonic acid, synthetic co-polymers, such as co-polymers of an alkoxy polyethylene glycol (meth)acrylate. Specific examples of co-binders are described in US 2004/0260050, US 2005/0003285 and US 2005/0123853. Printing plate precursors, the imageable layer of which includes a binder and optionally a co-binder according to this embodiment and described in more detail in US 2004/0260050, US 2005/0003285 and US 2005/0123853, optionally comprise a topcoat and an interlayer.

### Surfactants

Various surfactants may be added into the coating to allow or enhance the developability of the precursor; especially developing with a gum solution. These surfactants may be present in the photopolymerisable layer or in an optional other layer. Both polymeric and small molecule surfactants can be used. Nonionic surfactants are preferred. Preferred nonionic surfactants are polymers and oligomers containing one or more polyether (such as polyethylene glycol, polypropylene glycol, and copolymer of ethylene glycol and propylene glycol) segments. Examples of preferred nonionic surfactants are block copolymers of propylene glycol and ethylene glycol (also called block copolymer of propylene oxide and ethylene oxide); ethoxylated or propoxylated acrylate oligomers; and polyethoxylated alkylphenols and polyethoxylated fatty alcohols. The nonionic surfactant is preferably added in an amount ranging between 0.01 and 20% by weight of the coating, more preferably between 0.1 and 10% by weight of the coating, and most preferably between 0.5 and 5% by weight of the coating.

### Colorants

The photopolymerisable layer or an optional other layer of the coating may also comprise a colorant. After processing, at least part of the colorant remains on the hardened coating areas, and a visible image can be produced on the support by removing the coating, including the colorant, at the non-exposed areas. The colorant can be a dye or a pigment. Various types of pigments can be used such as organic pigments, inorganic pigments, carbon black, metallic powder pigments and fluorescent pigments. Organic pigments are preferred.

Specific examples of organic pigments include quinacridone pigments, quinacridonequinone pigments, dioxazine pigments, phthalocyanine pigments, anthrapyrimidine pigments, anthanthrone pigments, indanthrone pigments, flavanthrone pigments, perylene pigments, diketopyrrolopyrrole pigments, perinone pigments, quinophthalone pigments, anthraquinone pigments, thioindigo pigments, benzimidazolone pigments, isoindolinone pigments, azomethine pigments, and azo pigments.

Specific examples and more detailed information of pigments suitable as colorant in the current invention are described in EP 2 278 404 in paragraphs [0064] to [0068].

Typically, the amount of pigment in the coating may be in the range of about 0.005 g/m² to 2 g/m², preferably about 0.007 g/m² to 0.5 g/m², more preferably about 0.01 g/m² to 0.2 g/m², most preferably about 0.01 g/m² to 0.1 g/m².

The colorant can also be a dye. Any known dyes, such as commercially available dyes or dyes described in, for example, "Dye Handbook" (edited by the Organic Synthetic Chemistry Association, published in 1970) which are colored for the human eye, can be used as colorant in the photopolymerisable coating. Specific examples thereof are described in EP 2 278 404 in paragraph [0070].

Typically, the amount of dye in the coating may be in the range of about 0.005 g/m² to 2 g/m², preferably about 0.007 g/m² to 0.5 g/m², more preferably about 0.01 g/m² to 0.2 g/m², most preferably about 0.01 g/m² to 0.1 g/m².

The photopolymerisable layer or an optional other layer of the coating may include a printing-out agent, i.e. a compound which is capable of changing the color of the coating upon exposure. After image-wise exposing of the precursor, a visible image can be produced, hereinafter also referred to as "print-out image". The printing-out agent may be a compound as described in EP-A-1 491 356 paragraph [0116] to [0119] on page 19 and 20, and in US 2005/8971 paragraph [0168] to [0172] on page 17. Preferred printing-out agents are the compounds described in EP 1 765 592 from line 1 page 9 to line 27 page 20. More preferred are the IR-dyes as described in EP 1 736 312 from line 32 page 5 to line 9 page 32. The contrast of the image formed after image-wise exposure and processing is defined as the difference between the optical density at the exposed area to the optical density at the non-exposed area, and this contrast is preferably as high as possible. This enables the end-user to establish immediately whether or not the precursor has already been exposed and processed, to distinguish the different color selections and to inspect the quality of the image on the plate precursor. The contrast increases with increasing optical density in the exposed areas and/or decreasing optical density in the non-exposed areas. The optical density in the exposed area may increase with the amount and extinction coefficient of the colorant remaining in the exposed areas and the intensity of color formed by the printing-out agent. In the non-exposed areas it is preferred that the amount of colorant is as low as possible and that the intensity of color print-out agent is as low as possible. The optical density can be measured in reflectance using an optical densitometer, equipped with several filters (e.g. cyan, magenta, yellow). The difference in optical density in the exposed area and the non-exposed area has preferably a value of at least 0.3, more preferably at least 0.4, most preferably at least 0.5. There is no specific upper limit for the contrast value, but typically the contrast is not higher than 3.0 or even not higher than 2.0. In order to obtain a good visual contrast for a human observer the type of color of the colorant may also be important. Preferred colors for the colorant are cyan or blue colors, i.e. under blue color we understand a color that appears blue for the human eye.

### Other ingredients

The photopolymerizable layer or an optional other layer of the coating may also comprise an inhibitor. Particular inhibitors for use in the photopolymer coating are disclosed in US 6,410,205, EP 1 288 720 and EP 1 749 240. The photopolymerizable layer or an optional other layer of the coating may further comprise an adhesion promoting compound. More information on suitable adhesion promoting compounds are described in EP 1 788 434 in [0010]; in EP 2 855 152 and in WO2009/063024 (page 15, line 17 to page 16, line 30).

The coating may further comprise particles which increase the resistance of the coating against manual or mechanical damage. The particles may be inorganic particles, such as for example silica, alumina, iron oxides, magnesium carbonate, titanium oxide and calcium carbonate. The particles may be organic particles or fillers, such as for example polymer particles, waxes, carbon black and silicone resins. More information on suitable particles is described in for example US 7,108,956. The particles preferably have a particle size of about 0.01 to 15 µm, more preferably between 0.5 *µ*m and 7 *µ*m, most preferably between 1 *µ*m and 5 *µ*m. The particle size refers to the average particle size and may be measured by a laser diffraction particle analyzer such as the Coulter LS Particle Size Analyzer, e.g. the Coulter LS-230, commercially available by Beckman Coulter Inc. The average particle size is defined as the mean or median of the volume distribution of particle size. For obtaining a significant effect of improving the resistance of the coating against manual or mechanical damage, the spacer particles should extend the surface of the coating. The coating has preferably a layer thickness greater than 0.5 g/m², more preferably the layer thickness is comprised between 0.6 g/m² and 2.8 g/m². The particle size of the spacer particles is preferably comprised between one to two times the thickness of the coating.

Examples of inorganic spacer particles include silicium, titanium, aluminium, zinc, iron, chromium or zirconium containing particles, metal oxides or hydroxides thereof, aluminiumsilicates, and metal salts such as calcium carbonate, barium sulfate, barium titanate and strontium titanate.

Examples of organic spacer particles include optionally cross-linked polyalkyl(meth)acrylate such as polymethylmethacrylate, polystyrene, melamine, polyolefins such as polyethylene or polypropylene, halogenated polyolefins such as fluorinated polyolefins for example polytetrafluoroethylene, silicones such as cross-linked polysiloxane particles, or copolymers thereof. Examples of polysiloxane particles include cross-linked polyalkylsiloxanes such as polymethylsiloxane. Commercially available cross-linked polysiloxane particles are for example Tospearl from TOSHIBA SILICONE Co.,Ltd.

The ingredients may be present in the photopolymer layer or in an optional other layer of the coating.

### Protective layer

The coating may include on the photopolymerisable layer, a toplayer or protective overcoat layer which acts as an oxygen barrier layer including water-soluble or water-swellable binders. Printing plate precursors which do not contain a toplayer or protective overcoat layer are also referred to as overcoat-free printing plate precursors. In the art, it is well-known that low molecular weight substances present in the air may deteriorate or even inhibit image formation and therefore usually a toplayer is applied to the coating. However, as a toplayer should be easily removable during development, adhere sufficiently to the photopolymerisable layer or optional other layers of the coating and should preferably not inhibit the transmission of light during exposure, overcoat-free photopolymer printing plate precursors are desirable. Preferred binders which can be used in the toplayer are polyvinyl alcohol and the polymers disclosed in WO 2005/029190; US 6,410,205 and EP 1 288 720, including the cited references in these patents and patent applications. The most preferred binder for the toplayer is polyvinylalcohol. The polyvinylalcohol has preferably a hydrolysis degree ranging between 74 mol % and 99 mol %, more preferably between 88-98%. The weight average molecular weight of the polyvinylalcohol can be measured by the viscosity of an aqueous solution, 4 % by weight, at 20°C as defined in DIN 53 015, and this viscosity number ranges preferably between 3 and 26, more preferably between 3 and 15, most preferably between 3 and 10.

The coating thickness of the optional toplayer is preferably between 0.25 and 1.75 g/m², more preferably between 0.25 and 1.3 g/m², most preferably between 0.25 and 1.0 g/m². In a more preferred embodiment of the present invention, the optional toplayer has a coating thickness between 0.25 and 1.75 g/m² and comprises a polyvinylalcohol having a hydrolysis degree ranging between 74 mol % and 99 mol % and a viscosity number as defined above ranging between 3 and 26.

### Support

The support is preferably a grained and anodized aluminium support, well known in the art. Suitable supports are for example disclosed in EP 1 843 203 (paragraphs [0066] to [0075]). The surface roughness, obtained after the graining step, is often expressed as arithmetical mean center-line roughness Ra (ISO 4287/1 or DIN 4762) and may vary between 0.05 and 1.5 µm. The aluminum substrate of the current invention has preferably an Ra value below 0.45 µm, more preferably below 0.40 µm and most preferably below 0.30 µm. The lower limit of the Ra value is preferably about 0.1 µm. More details concerning the preferred Ra values of the surface of the grained and anodized aluminum support are described in EP 1 356 926. By anodising the aluminum support, an Al₂O₃ layer is formed and the anodic weight (g/m² Al₂O₃ formed on the aluminum surface) varies between 1 and 8 g/m². The anodic weight is preferably ≥ 3 g/m², more preferably ≥ 3.5 g/m² and most preferably ≥ 4.0 g/m².

The grained and anodized aluminium support may be subjected to so-called post-anodic treatments, for example a treatment with polyvinylphosphonic acid or derivatives thereof, a treatment with polyacrylic acid, a treatment with potassium fluorozirconate or a phosphate, a treatment with an alkali metal silicate, or combinations thereof. Alternatively, the support may be treated with an adhesion promoting compound such as those described in EP 1 788 434 in [0010] and in EP 2 855 152. For a precursor optimized to be used without a pre-heat step it is preferred to use a grained and anodized aluminium support without any post-anodic treatment.

Besides an aluminium support, a plastic support, for example a polyester support, provided with one or more hydrophilic layers as disclosed in for example EP 1 025 992 may also be used.

The lithographic printing plate precursor of the present invention can be prepared by (i) applying on a support as described above the coating as described above and (ii) drying the precursor.

### Method for making a printing plate

According to the present invention there is also provided a method for making a negative-working lithographic printing plate comprising the steps of imagewise exposing the printing plate precursor followed by developing the imagewise exposed precursor so that the non exposed areas are dissolved in the developer solution. Optionally, after the imaging step, a heating step is carried out to enhance or to speed-up the polymerization and/or crosslinking reaction.

### Exposure

The image-wise exposing step can be carried out by a laser. Preferably, the image-wise exposing step is carried out off-press in a platesetter, i.e. an exposure apparatus suitable for image-wise exposing the precursor with a laser such as a laser diode, emitting around 830 nm, a Nd YAG laser, emitting around 1060 nm, a violet laser, emitting around 400 nm, or a gas laser such as an Ar laser, or with a digitally modulated UV-exposure set-up, using e.g. digital mirror devices, or by a conventional exposure in contact with a mask. In a preferred embodiment of the present invention, the precursor is image-wise exposed by a laser emitting violet light.

### Heating step

During the optional heating step the plate precursor is heated, preferably at a temperature of about 80°C to 150°C and preferably during a dwell time of about 5 seconds to 1 minute. The preheating step is preferably carried out in a preheating unit which is preferably provided with heating elements such as IR-lamps, UV-lamps, heated air, a heated metal roll, etc.

### Washing step

After the exposing step or, when a preheating step is present, after the preheating step, the precursor may be washed in a prewashing station, whereby at least part of the toplayer, if present, can be removed by supplying a wash liquid, i.e. water or an aqueous solution, to the coating of the precursor. The washing liquid is preferably water, more preferably tap water. More details concerning the wash step are described in EP 1 788 434 in [0026].

### Development

After the exposure step, the optional heating step and the optional prewashing step, the precursor is preferably developed by means of immersing the precursor in a developing solution. The developing step is preferably carried out off-press with an aqueous alkaline developing solution (typically with an alkaline developer having a pH > 10) or a gum solution. During the development step, the non-exposed areas of the image-recording layer are at least partially removed without essentially removing the exposed areas. The processing liquid can be applied to the plate e.g. by rubbing with an impregnated pad, by dipping, immersing, (spin-)coating, spraying, pouring-on, either by hand or in an automatic processing apparatus. The treatment with a processing liquid may be combined with mechanical rubbing, e.g. by a rotating brush. The developed plate precursor can, if required, be post-treated with rinse water, a suitable correcting agent or preservative as known in the art. During the development step, any water-soluble protective layer present is preferably also removed. The development is preferably carried out at temperatures between 20 and 40 °C in automated processing units as customary in the art. More details concerning the development step can be found in for example EP 1 614 539 in [42] to [43].

The development step with an aqueous alkaline developing solution may be followed by a rinsing step and/or a gumming step.

Alternatively, the development step can be carried out by applying a gum solution thereby removing the non-exposed areas of the photopolymerisable layer from the support and gumming the plate in a single step. Preferably, the gumming unit is mechanically coupled to the platesetter by conveying means wherein the precursor is shielded from ambient light. Development in a gumming station comprising at least one gumming unit is for example described in WO 2007/057348 on page 40 line 34 to page 44 line 14.

A gum solution is typically an aqueous liquid which comprises one or more surface protective compounds that are capable of protecting the lithographic image of a printing plate against contamination, e.g. by oxidation, fingerprints, fats, oils or dust, or damaging, e.g. by scratches during handling of the plate. Suitable examples of such compounds are film-forming hydrophilic polymers or surfactants. The layer that remains on the plate after treatment with the gum solution preferably comprises between 0.005 and 20 g/m² of the surface protective compound, more preferably between 0.010 and 10 g/m², most preferably between 0.020 and 5 g/m². More details concerning the surface protective compounds in the gum solution can be found in WO 2007/057348 page 9 line 3 to page 11 line 6.

The gum solution preferably has a pH value between 3 and 11, more preferably between 4 and 10, even more preferably between 5 and 9, and most preferably between 6 and 8. A suitable gum solution is described in for example EP 1 342 568 in [0008] to

The viscosity of the gum solution can be adjusted to a value of e.g. between 1.7 and 5 mPa.s, by adding viscosity increasing compounds, such as poly(ethylene oxide) or polyvinylalcohol, e.g. having a molecular weight between 10⁴ and 10⁷. Such compounds can be present in a concentration of 0.01 to 10 g/l.

The gum solution may further comprise an inorganic salt, an anionic surfactant, a wetting agent, a chelate compound, an antiseptic compound, an anti-foaming compound and/or an ink receptivity agent and/or combinations thereof. More details about these additional ingredients are described in WO 2007/057348 page 11 line 22 to page 14 line 19.

Alternatively, the development step can be carried out on press by mounting the exposed precursor on a plate cylinder of a lithographic printing press and rotating the plate cylinder while feeding dampening liquid and/or ink to the coating.

After the processing step the plate may be dried in a drying unit. In a preferred embodiment the plate is dried by heating the plate in the drying unit which may contain at least one heating element selected from an IR-lamp, an UV-lamp, a heated metal roller or heated air. In a preferred embodiment of the present invention, the plate is dried with heated air as known in the drying section of a classical developing machine.

After drying the plate, the plate can optionally be heated in a baking unit. More details concerning the heating in a baking unit can be found in WO 2007/057348 page 44 line 26 to page 45 line 20.

The printing plate thus obtained can be used for conventional, so-called wet offset printing, in which ink and an aqueous dampening liquid is supplied to the plate. Another suitable printing method uses a so-called single-fluid ink without a dampening liquid. Suitable single-fluid inks have been described in US 4,045,232; US 4,981,517 and US 6,140,392. In a most preferred embodiment, the single-fluid ink comprises an ink phase, also called the hydrophobic or oleophilic phase, and a polyol phase as described in WO 00/32705.

### EXAMPLES

### I. Synthesis of the inventive photoinitiators HABI-PEG 1 to 4, 18, 12 and 13.

A11 materials used were readily available from standard sources such as Sigma-Aldrich (Belgium) and Acros (Belgium) unless otherwise specified.

### 1. Synthesis of PEG-HABI 1, PEG-HABI 2 and PEG-HABI 3

### Synthesis of 4,4'-dihydroxybenzil

45 g (0.166 mol) 4,4-dimethoxybenzil was mixed with 100 g pyridine hydrochloride (0.865 mol) and the mixture was heated to 180°C. The reaction mixture was kept at 180°C for 14 hours. The mixture was allowed to cool down and then poured into 500 ml water. The mixture was extracted with 250 ml ethyl acetate. The organic fraction was washed three times with 100 ml brine and dried over MgSO₄. The solvent was evaporated under reduced pressure and 4,4'-dihydroxybenzil was isolated as a pale yellow powder.

35.2 g (yield: 87.5 %) of 4,4'-dihydroxybenzil was isolated and used without further purification.

### Synthesis of mesylated monomethoxy polyethylene glycol

The procedure for synthesis of various mesylated monomethoxy polyethylene glycols is illustrated starting from monomethoxy polyethylene glycol 750. Mesylated monomethoxy polyethylene glycol 350 and mesylated monomethoxy polyethylene glycol 550 were prepared using the same synthetic procedure.

225 g (0.3 mol) monomethoxy polyethylene glycol 750 was dissolved in 850 ml methylene chloride. 46.52 g (0.36 mol) diisopropyl ethyl amine was added and the mixture was cooled to 0°C. 41.23 g (0.36 mol) mesyl chloride was dissolved in 150 ml methylene chloride and added dropwise to the reaction mixture over 45 minutes while keeping the temperature between 5 and 7°C. The reaction was allowed to continue at room temperature for four hours. The reaction mixture was extracted three times with 150 ml brine. The methylene chloride fraction was dried over MgSO₄. The solvent was evaporated under reduced pressure and mesylated monomethoxy polyethylene glycol 750 was isolated as a yellow oil. 233.4 g (yield = 92%) of mesylated monomethoxy polyethylene glycol 750 was isolated and used without further purification.

### Synthesis of monomethoxy ethoxylated 4,4'-dialkoxybenzil

### 3.1 Starting from mesylated monomethoxy polyethylene glycol 750

29.84 g (0.036 mol) of mesylated monomethoxy polyethylene glycol 750 and 3.6 g (0.015 mol) 4,4'-dihydroxybenzil were dissolved in 300 ml acetonitrile. 12.44 g (0.09 mol) K₂CO₃ was added and the mixture was refluxed for 18 hours. The mixture was allowed to cool down to room temperature. The salts were removed by filtration and the solvent was removed under reduced pressure. The isolated oil was directly used in the cyclisation to the imidazole.

### 3.2 Starting from mesylated monomethoxy polyethylene glycol 350

45.98 g (0.11 mol) of mesylated monomethoxy polyethylene glycol 350 and 12.1 g (0.05 mol) 4,4'-dihydroxybenzil were dissolved in 300 ml acetonitrile. 41.63 g (0.09 mol) K₂CO₃ was added and the mixture was refluxed for 18 hours. An additional 4.5 g (0.011 mol) of mesylated monomethoxy polyethylene glycol 350 was added and the reaction was allowed to continue for 48 hours at 80°C. The mixture was allowed to cool down to room temperature. The salts were removed by filtration and the solvent was removed under reduced pressure. The residue was dissolved in 300 ml methylene chloride and extracted three times with 250 ml 1N NaOH. The organic fraction was isolated and dried over Na₂SO₄. The solvent was removed under reduced pressure and the ethoxylated benzil derivative was purified by preparative column chromatography on a Prochrom LC80 column, using Kromasil C18 100 Å 10 *µ*m as stationary phase and methanol/ammonium acetate (0.2M) 70/30 as eluent. 5.2 g of the ethoxylated benzil derivative was isolated (TLC on Reveleris RP C18 plates supplied by Grace using methanol/1 M NaCl 80/20 as eluent; R_{f}: 0.48)

### 3.3 Starting from mesylated monomethoxy polyethylene glycol 550

23.22 g (0.036 mol) of mesylated monomethoxy polyethylene glycol 550 and 3.6 g (0.015 mol) 4,4'-dihydroxybenzil were dissolved in 300 ml acetonitrile. 12.44 g (0.09 mol) K₂CO₃ was added and the mixture was refluxed for 18 hours. The mixture was allowed to cool down to room temperature. The salts were removed by filtration and the solvent was removed under reduced pressure. The ethoxylated benzil derivative was purified by preparative column chromatography, on a Prochrom LC80 column, using Kromasil C18 100 Å 10 *µ*m as stationary phase and methanol/ammonium acetate (0.2M) 70/30 as eluent. 6.56 g of the ethoxylated benzil derivative was isolated (TLC on Reveleris RP C18 plates supplied by Grace using methanol/1 M NaCl 80/20 as eluent; R_{f}: 0.38).

### Synthesis of monomethoxy ethoxylated 2-(2-chlorophenyl)-4,5-bis(4-hydroxyphenyl)-1-H-imidazole

### 4.1 Monomethoxy polyethylene glycol 750 as ethoxylating fragment

34 g (19.9 mmol) of the crude ethoxylated 4,4'-dialkoxybenzil and 3.48 g (24.8 mmol) 2-chloro-benzaldehyde were dissolved in 51.26 g acetic acid. 7.6 g (99.5 mmol) ammonium acetate was added and the mixture was heated to 80°C. The reaction was allowed to continue for 18 hours at 80°C. The reaction mixture was allowed to cool down to room temperature and acetic acid was removed under reduced pressure. The crude ethoxylated imidazole derivative was purified by preparative column chromatography, on a Prochrom LC80 column, using Kromasil C18 100 Å 10 *µ*m as stationary phase and methanol / ammonium acetate (0.2M) 75/25 as eluent. 13.3 g of the ethoxylated imidazole derivative was isolated (TLC on Reveleris RP C18 plates supplied by Grace using methanol/1 M NaCl 80/20 as eluent: R_{f} : 0.42)

### 4.2 monomethoxy polyethylene glycol 350 as ethoxylating fragment

5.2 g (5.5 mmol) of the crude ethoxylated 4,4'-dialkoxybenzil and 1.63 g (11.6 mmol) 2-chloro-benzaldehyde were dissolved in 25 g acetic acid. 3.58 g (46.5 mmol) ammonium acetate was added and the mixture was heated to 80°C. The reaction was allowed to continue for 18 hours at 80°C. The reaction mixture was allowed to cool down to room temperature and acetic acid was removed under reduced pressure. The crude ethoxylated imidazole derivative was purified by preparative column chromatography on a Prochrom LC80 column, using Kromasil C18 100 Å 10 *µ*m as stationary phase and methanol / ammonium acetate (0.2M) 73/27 as eluent. 3.27 g of the ethoxylated imidazole derivative was isolated (TLC on Reveleris RP C18 plates supplied by Grace using methanol/1 M NaCl 80/20 as eluent: R_{f} : 0.24)

### 4.3 monomethoxy polyethylene glycol 550 as ethoxylating fragment

6.56 g (5 mmol) of the crude ethoxylated 4,4'-dialkoxybenzil and 0.88 g (6.2 mmol) 2-chloro-benzaldehyde were dissolved in 13.5 g acetic acid. 1.9 g (25 mmol) ammonium acetate was added and the mixture was heated to 80°C. The reaction was allowed to continue for 18 hours at 80°C. The reaction mixture was allowed to cool down to room temperature and acetic acid was removed under reduced pressure. The crude ethoxylated imidazole derivative was purified by preparative column chromatography on a Prochrom LC80 column, using Kromasil C18 100 Å 10 *µ*m as stationary phase and methanol / ammonium acetate (0.2M) 72/28 as eluent. 1.92 g of the ethoxylated imidazole derivative was isolated (TLC on Reveleris RP C18 plates supplied by Grace using methanol/1 M NaCl 80/20 as eluent, R_{f} : 0.32)

### Synthesis of PEG-HABI-1

A solution in water was prepared containing 0.3 g (1.1 mmol) tetrabutyl ammonium chloride, 1.7 g (42.9 mmol) sodium hydroxide and 2.14g (6.6 mmol) K₃Fe(CN)₆ in 100 ml water. 4 g (2.2 mmol) of the ethoyxylated imidazole derivative derived from monomethoxy polyethylene glycol 750 was dissolved in 100 ml methylene chloride and both solutions were mixed while stirring. The mixture was refluxed for six hours. The mixture was allowed to cool down to room temperature and the organic fraction was isolated. The aqueous fraction was extracted three times with 100 ml methylene chloride and the pooled organic fractions were dried over Na₂SO₄. The solvent was removed under reduced pressure and PEG-HABI-1 was isolated by preparative column chromatography on a Prochrom LC80 column, using Kromasil C18 100 Å 10 *µ*m as stationary phase and methanol / ammonium acetate (0.2M) 72/28 as eluent. 2.1 g of the ethoxylated bisimidazole derivative was isolated (TLC on Reveleris RP C18 plates supplied by Grace using methanol/1 M NaCl 85/15 as eluent: R_{f} : 0.3)

### Synthesis of PEG-HABI-2

A solution in water was prepared containing 0.41 g (1.5 mmol) tetrabutyl ammonium chloride, 2.43 g (58.5 mmol) sodium hydroxide and 2.96 g (9 mmol) K₃Fe(CN)₆ in 100 ml water. 3.27 g (3 mmol) of the ethoyxylated imidazole derivative, derived from monomethoxy polyethylene glycol 350 was dissolved in 100 ml methylene chloride and both solutions were mixed while stirring. The mixture was refluxed for six hours. The mixture was allowed to cool down to room temperature and the organic fraction was isolated. The aqueous fraction was extracted three times with 100 ml methylene chloride and the pooled organic fractions were dried over Na₂SO₄. The solvent was removed under reduced pressure and PEG-HABI-2 was isolated by preparative column chromatography on a Prochrom LC80 column, using Kromasil C18 100 Å 10 *µ*m as stationary phase and methanol / ammonium acetate (0.2M) 72/28 as eluent. 2.14 g of the ethoxylated bisimidazole derivative was isolated (TLC on Reveleris RP C18 plates supplied by Grace using methanol/1 M NaCl 85/15 as eluent : R_{f} : 0.32)

### Synthesis of PEG-HABI-3

A solution in water was prepared containing 0.18 g (0.65 mmol) tetrabutyl ammonium chloride, 1.0 g (25 mmol) sodium hydroxide and 1.284 g (3.9 mmol) K₃Fe(CN)₆ in 100 ml water. 1.92 g (1.3 mmol) of the ethoyxylated imidazole derivative, derived from monomethoxy polyethylene glycol 550 was dissolved in 100 ml methylene chloride and both solutions were mixed while stirring. The mixture was refluxed for six hours. The mixture was allowed to cool down to room temperature and the organic fraction was isolated. The aqueous fraction was extracted twice times with 50 ml methylene chloride and the pooled organic fractions were dried over Na₂SO₄. The solvent was removed under reduced pressure and PEG-HABI-3 was isolated by preparative column chromatography on a Prochrom LC80 column, using Kromasil C18 100 Å 10 *µ*m as stationary phase and methanol / ammonium acetate (0.2M) 72/28 as eluent. 1.29 g of the ethoxylated bisimidazole derivative was isolated (TLC on Reveleris RP C18 plates supplied by Grace using methanol/1 M NaCl 85/15 as eluent : R_{f} : 0.3)

### 2. Synthesis of PEG-HABI 4

### Tosylation of terta-ethylene glycol monomethyl ether

150.41 g (0.79 mol) tosyl chloride was dissolved in 400 ml methylene chloride. 156.41 g (0.752 mol) tetraethylene glycol monomethyl ether was added and the mixture was cooled to 0°C. 79.74 g (0.79 mol) triethyl amine in 100 ml methylene chloride was added while the temperature was kept below 10°C. The reaction was allowed to continue for three hours at room temperature. The reaction mixture was extracted twice with 300 ml 5N NaOH and twice with 300 ml water. The organic fraction was dried over Na₂SO₄ and evaporated under reduced pressure. The crude product was purified using preparative column chromatography on a Prochrom LC80 column, using Kromasil Si60A 10 *µ*m as statoionary phase and methylene chloride/ethyl acetate 35/65 as eluent. 108 g of the tosylated tertaethylene glycol monomethyl ether was isolated as a slightly colored oil (y : 39.6 %) (TLC analysis on TLC Silicagel 60F₂₅₄ supplied by Merck, methylene chloride/ethyl acetate 35/65 as eluent : R_{f} = 0.45).

### The alkylation of 4,4'-dihydroxybenzil

To 375 ml dimethyl formamide, 12.10 g (50 mmol) 4,4'-dihydroxybenzil (synthesis see above), 39.65 g (0.113 mol) tosylated tertaethylene glycol monomethyl ether, 8.34 g (30 mmol) tetrabutyl ammonium chloride and 16.5 g (0.12 mol) potassium carbonate were added. The reaction mixture was heated to 90°C for two and a half hours. The reaction mixture was allowed to cool down to room temperature. 1250 ml water was added to the reaction mixture and the mixture was extracted twice with 100 ml methyl t-butyl ether and twice with 100 ml ethylacetate. The pooled organic fractions were dried over Na₂SO₄ and evaporated under reduced pressure. The crude alkylated 4,4-dihydroxybenzil was purified by preparative column chromatography on a Prochrom LC 80 column, using Kromasil C18 100Å 10 *µ*m as stationary phase and methanol / 0.2 M ammonium acetate 70/30 as eluent. 8.88 g of the alkylated 4,4-dihydroxybenzil was isolated as a yellow oil (y : 28%) (TLC analysis on Reveleris RP C18 plates supplied by Grace, methanol/1 M NaCl 80/20 as eluent, R_{f} = 0.48).

### Ring closure with 2-chlorobenzaldehyde

8.8 g (14 mmol) of the alkylated 4,4'-dihydroxybenzil, 5.43 g (70.5 mmol) ammonium acetate and 1.98 g (14 mmol) 2-chlorobenzaldehyde were dissolved in 25.4 ml acetic acid. The mixture was heated to 80°C for 20 hours. The reaction mixture was allowed to cool down to room temperature and the solvent was removed under reduced pressure. The residue was dissolved in 125 ml methylene chloride and extracted twice with 75 ml 2N NaOH and twice with 100 ml water. The organic fraction was dried over Na₂SO₄ and the solvent was evaporated under reduced pressure. 7.9 g of the crude triaryl imidazole was isolated and used without further purification.

### Synthesis of PEG-HABI 4

A solution of 1.47 g (5.3 mmol) tetrabutylammonium chloride, 8.27 g (0.207 mol) sodium hydroxide and 10.46 g (31.8 mmol) K₃Fe(CN)₆ in 150 ml water was prepared. 7.9 g (10.6 mmol) of the ethoxylated triaryl imidazole dissolved in 150 ml methylene chloride was added and the mixture was refluxed for three hours. The reaction mixture was allowed to cool down to room temperature. The organic fraction was isolated. The aqueous fraction was extracted twice with 75 ml methylene chloride. The pooled organic fractions were dried over Na₂SO₄ and evaporated under reduced pressure. The crude PEG-HABI 4 was purified by preparative column chromatography on a GraceResolv column, supplied by Grace, using a gradient elution from methylene chloride to methylene chloride/methanol 97/3. 4.45 g of PEG-HABI 4 was isolated (y : 56.5 %) (TLC analysis on Reveleris RP C18 plates supplied by Grace, methanol/1 M NaCl 90/10 as eluent, R_{f} = 0.44). PEG-HABI 4 was analyzed using HPLC in combination with an AmaZon SL mass spectrometer (supplied by Brüker Daltonics). An Altima C18 column (150x3 mm, 5*µ*m), supplied by Alltech was used. A gradient elution from water to acetonitrile over 13 minutes, followed by an isocratic elution for 17 minutes at a flow rate of 0.5 ml/min and a temperature of 40°C was used. A sample of 2 mg PEG-HABI 4 in 20 ml acetonitrile was prepared. 5 *µ*l of this sample was injected. The structure of PEG-HABI 4 was confirmed and the sample had a purity of 97.6 % based on area percentages.

### 3. Synthesis of PEG-HABI-18

PEG-HABI-18 has been synthesized following the same procedure as described above for PEG-HABI 1 to PEG-HABI 4.

### 4. Synthesis of PEG-HABI 12

### The alkylation of salicaldehyde

24.42 g (0.2 mol) salicaldehyde was dissolved in 300 ml methanol. 11.22 g (0.2 mol) KOH was added and the mixture was stirred for 45 minutes. The solvent was removed under reduced pressure. The isolated potassium salt of salicaldehyde was suspended in 300 ml dimethyl formamide. A solution of 72.88 g (0.2 mol) of tosylated tetratethylene glycol monomethyl ether in 50 ml dimethylformamide was added. The reaction mixture was heated to 100°C and the reaction was allowed to continue for 18 hours at 100°C. The reaction mixture was allowed to cool down to room temperature and 500 ml methylene chloride was added. The mixture was extracted two times with 300 ml 2N NaOH and two times with 300 ml water. The organic fraction was dried over Na₂SO₄ and evaporated under reduced pressure. 48.7 g of the alkylated salicaldehyde was isolated and used without further purification.

### Cyclisation with benzyl to the triaryl imidazole

46.82 g (0.15 mol) of the alkylated salicaldehyde, 57.81 g (0.75 mol) ammonium acetate and 25.21 g g (0.12 mol) benzil were added to 270 g acetic acid. The mixture was heated to 80°C and the reaction was allowed to continue for 24 hours at 80°C. The reaction mixture was allowed to cool down to room temperature and the solvent was removed under reduced pressure. The crude triaryl imidazole was used without further purification.

### Synthesis of PEG-HABI 12

25.10 g of the crude triaryl imidazole was dissolved in 400 ml methylene chloride. This solution was added to a solution of 6.94 g (25 mmol) tetrabutyl ammonium chloride, 78 g (1.95 mol) sodium hydroxide and 98.77 g (0.3 mol) K₃Fe(CN)₃ in 400 ml water. The mixture was refluxed for four hours. The reaction mixture was allowed to cool down to room temperature and the organic fraction was isolated. The aqueous layer was extracted twice with 125 ml methylene chloride. The pooled methylene chloride fractions were dried over Na₂SO₄ and evaporated under reduced pressure. PEG-HABI 12 was purified by preparative column chromatography first on a GraceResolve column, supplied by Grace, using a gradient elution from methylene chloride to methylene chloride / methanol 93/7, followed by purification on a Prochrom LC 80 column, using Kromasil C18 100Å 10 *µ*m as stationary phase and methanol /0.2 M ammonium acetate as eluent. 2.73 g (y : 10.9%) of PEG-HABI 12 was isolated. (TLC analysis on Reveleris RP C18 plates supplied by Grace, methanol/1 M NaCl 80/20 as eluent, R_{f} = 0.17). PEG-HABI 12 was analyzed using HPLC in combination with an AmaZon SL mass spectrometer (supplied by Brüker Daltonics). An Altima C18 column (150x3 mm, 5*µ*m), supplied by Alltech was used. A gradient elution from water to acetonitrile over 13 minutes, followed by an isocratic elution for 17 minutes at a flow rate of 0.5 ml/min and a temperature of 40°C was used. A sample of 2 mg PEG-HABI 12 in 20 ml acetonitrile was prepared. 5 *µ*l of this sample was injected. The structure of PEG-HABI 4 was confirmed and the sample had a purity of 80.1 % based on area percentages. The starting triaryl imidazole was the main contaminant identified in the product (15 area %). PEG-HABI 12 was used in the evaluations without further purification.

### 5. Synthesis of PEG-HABI 13

### Synthesis of 2-(2-chloro-5-nitro-phenyl)-4,5-diphenyl-1H-imidazole:

37.5 g (0.2 mol) 2-chloro-5-nitro-benzaldehyde, 46.25 g (1 mol) ammonium acetate and 42.05 (0.2 mol) benzil were suspended in 360 g acetic acid. The reaction mixture was heated to 80°C and the reaction was allowed to continue for 20 hours at 80°C. The reaction mixture was allowed to cool down to room temperature and was added to 1000 g of an ice/water mixture. The crude 2-(2-chloro-5-nitro-phenyl)-4,5-diphenyl-1H-imidazole was isolated by filtration and dried. The crude 2-(2-chloro-5-nitro-phenyl)-4,5-diphenyl-1H-imidazole was dissolved in a mixture of 500 ml acetone, 100 ml methanol and 20 ml dimethylacetamide at 50°C. The mixture was added to 1000 g of an ice/water mixture. 2-(2-chloro-5-nitro-phenyl)-4,5-diphenyl-1H-imidazole was isolated by filtration and dried. 68 g (y : 90.5%) of 2-(2-chloro-5-nitro-phenyl)-4,5-diphenyl-1H-imidazole was isolated(m.p. : 220-222°C). 2-(2-chloro-5-nitro-phenyl)-4,5-diphenyl-1H-imidazole was analyzed with LC-MS, using HPLC in combination with an AmaZon SL mass spectrometer (supplied by Brüker Daltonics). An Altima C18 column (150x3 mm, 5*µ*m), supplied by Alltech was used. A gradient elution from water to acetonitrile over 13 minutes, followed by an isocratic elution for 17 minutes at a flow rate of 0.5 ml/min and a temperature of 40°C was used. A sample of 2 mg 2-(2-chloro-5-nitro-phenyl)-4,5-diphenyl-1H-imidazole in 20 ml acetonitrile was prepared. 5 *µ*l of this sample was injected. The structure of 2-(2-chloro-5-nitro-phenyl)-4,5-diphenyl-1H-imidazole was confirmed and the sample had a purity of 98.3 % based on area percentages.

### Synthesis of 2-(2-chloro-5-amino-phenyl)-4,5-diphenyl-1H-imidazole

52 g (0.138 mol) 2-(2-chloro-5-nitro-phenyl)-4,5-diphenyl-1H-imidazole and 156.1 g tin(II)chloride dihydrate were added to 600 ml methanol and the mixture was refluxed for two hours. The methanol was evaporated under reduced pressure and 500 ml of a 10 w% solution of sodium hydroxide was added. The mixture was extracted with 500 ml ethyl acetate. The organic fraction was isolated, filtered over Cellite, washed twice with 300 ml water and dried over MgSO₄. The solvent was evaporated under reduced pressure and the residue was dried. 42.2 g (y : 88.5%) of the crude 2-(2-chloro-5-amino-phenyl)-4,5-diphenyl-1H-imidazole was isolated. 2-(2-chloro-5-amino-phenyl)-4,5-diphenyl-1H-imidazole was used without further purification.

### Coupling with 2-[2-(2-methoxyethoxy)ethoxy]acetic acid

2.69 g (15.1 mmol) 2-[2-(2-methoxyethoxy)ethoxy] acetic acid was dissolved in 30 ml dimethyl acetamide. 3.0 g (18.7 mmol) carbodiimidazole was added and the reaction was allowed to continue for 1 hour at room temperature. 5.0 g (14.4 mmol) 2-(2-chloro-5-amino-phenyl)-4,5-diphenyl-1H-imidazole was added and the reaction was allowed to continue for 48 hours at room temperature. The solvent was removed under reduced pressure and the ethoxylated triaryl imidazole was isolated by preparative column chromatography on a Grace Resolve RS80 column, using a gradient elution from methylene chloride to methylene chloride/ethyl acetate 50/50. The isolated compound was redissolved in 10 ml methylene chloride and crystallized by the addition of hexane. 2.69 g (y : 37 %) of the ethoxylated triaryl imidazole was isolated (TLC analysis on TLC Silicagel 60 F₂₅₄, supplied by Merck, eluent methylene chloride/ethyl acetate 50/50, R_{f} : 0.27).

### Synthesis of PEG HABI-13

A solution of 2.2 g (7.9 mmol) tetrabutyl ammonium chloride (TBAC1) and 12.32 g sodium hydroxide in 150 ml water was prepared. 15.60 g (47.4 mmol) potassium hexacyanoferrate was added. The mixture was stirred at room temperature. To this mixture, a solution of 8 g (15.8 mmol) of the ethoxylated triaryl imidazole in 150 ml methylene chloride was added. The mixture was refluxed for four hours while intensively stirring. The organic fraction was isolated, washed twice with 120 ml water, dried over MgSO₄ and evaporated under reduced pressure, yielding the crude PEG HABI-13. The crude HABI-13 was purified using preparative column chromatography on a Prochrom LC80 column, using Kromasil Si 60Å 10 µm as stationary phase and ethyl acetate/methanol 96/4 as eluent. 3 g (y : 38 %) of HABI-13 was isolated. HABI-13 was analyzed using LC-MS as described in step 1. The structure of HABI-13 was confirmed and the sample had a purity of 98 % based on area percentages.

### II. Printing plates PP-01 to PP-05

### Preparation of the aluminium support S-01

A 0.3 mm thick aluminum foil was degreased by spraying with an aqueous solution containing 26 g/l NaOH at 65°C for 2 seconds and rinsed with demineralised water for 1.5 seconds. The foil was then electrochemically grained during 10 seconds using an alternating current in an aqueous solution containing 15g/l HCl, 15g/l SO42-ions and 5g/l Al3+ ions at a temperature of 37°C and a current density of about 100 A/dm2. Afterwards, the aluminum foil was desmutted by etching with an aqueous solution containing 5.5 g/l NaOH at 36°C for 2 seconds and rinsed with demineralised water for 2 seconds. The foil was subsequently subjected to anodic oxidation during 15 seconds in an aqueous solution containing 145 g/l of sulfuric acid at a temperature of 50°C and a current density of 17 A/dm2, then washed with demineralised water for 11 seconds and post-treated for 3 seconds (by spray) with a solution containing 2.2 g/l PVPA at 70°C, rinsed with demineralised water for 1 seconds and dried at 120°C for 5 seconds.

The support thus obtained was characterised by a surface roughness Ra of 0.35-0.4 *µ*m (measured with interferometer NT1100) and had an anodic weight of 3.0 g/m².

### Preparation of the printing plates PP-01 to PP-05

### Coating

The printing plate precursors were produced by coating onto the above described support S-01 the photopolymerisable layers PL-01 to PL-05 as defined in Table 1 dissolved in a mixture of 35% by volume of MEK and 65% by volume of Dowanol PM (1-methoxy-2-propanol, commercially available from DOW CHEMICAL Company). The coating solutions PL-01 to PL-05 were applied at a wet coating thickness of 26 µm and then dried at 120°C for 1 minute in a circulation oven. After drying, a dry coating weight of 1.2 to 1.5 g/m² was obtained.

**Table 1: dry coating weight of the photopolymerisable layers PL-01 to PL-05**

| **INGREDIENT** | **PL**-**01** | **PL-02** | **PL-03** | **PL-04** | **PL**-**5** |
|---|---|---|---|---|---|
| (mg/m²) | | | | | |
| Edaplan LA411 (1) | 1,2 | = | = | = | = |
| Hydroxyquinone | 0,6 | = | = | = | = |
| Sensitizer (2) | 51,6 | = | = | = | = |
| MBT (3) | 7,0 | = | = | = | = |
| FST 426 (4) | 389,2 | = | = | = | = |
| KOMA 30 (5) | 279,0 | = | = | = | = |
| Mono Z1620 (6) | 297,4 | = | = | = | = |
| Pig disp (7) | 96,0 | = | = | = | = |
| HABI 1 (8) | 54,6 | - | - | - | 54.6 |
| PEG-HABI-2 (9) (PEG-OMe 350) | - | 174,7 | - | - | - |
| PEG-HABI-3 (9) (PEG-OMe 550) | - | - | 234,8 | - | - |
| PEG-HABI-1 (9) (PEG-OMe 750) | - | - | - | 305,7 | |
| MPEG350 (10) | - | - | - | - | 120,1 |
| **Dry coating weight** | 1176,60 | 1296,70 | 1356,8 | 1427,7 | 1296,7 |
| (1) Edaplan LA411, commercially available from Munzing Chemie: | | | | | |
| 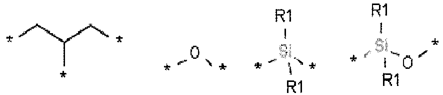 | | | | | |
| 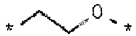 | | | | | |
| (2) Violet sensitizer mixture; synthesized as described in the Examples of WO2008/145528: preparation of SSMIX 5; containing the following compounds: | | | | | |
| 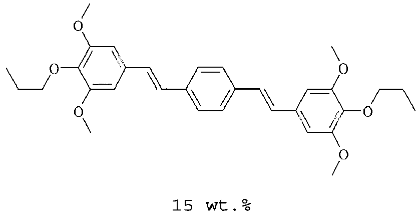 | | | | | |
| 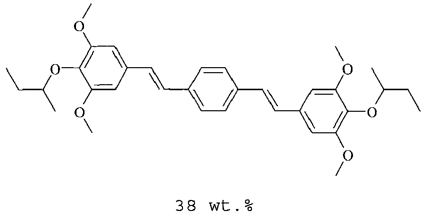 | | | | | |
| 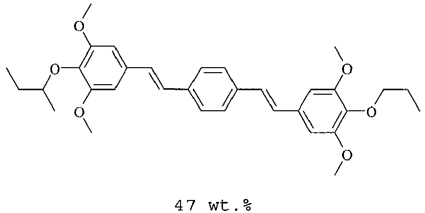 | | | | | |
| (3) MBT, 2-mercaptobenzthiazole, commercially available from Agfa Graphics NV; FST 426R™ is a reaction product from 1 mole of 2,2,4-trimethylhexamethylenediisocyanate and 2 moles of hydroxyethyl-methacrylate; | | | | | |
| (4) Polyvinylbutyral based binder, commercially available from Clariant; | | | | | |
| (5) Mono Z1620, monomer, commercially available from Clariant; | | | | | |
| (6) MCB-2 Comparative Photoinitiator, commercially available from Hamford Research; | | | | | |
| (7) Pig-disp contains in a 50/50 ratio PB60 commercially available from CLARIANT and KOMA 30 commercially available from Clariant; | | | | | |
| (8) HABI-1, comparative Photoinitiator, commercially available from Hodogaya Chemical | | | | | |
| 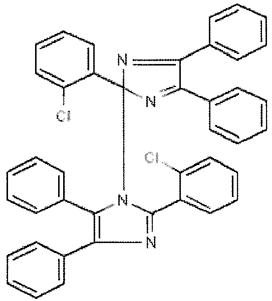 | | | | | |
| (9) Inventive photoinitiators, see Examples above; | | | | | |
| (10) Monomethoxy polyethylene glycol 350. | | | | | |

### Top layer OC-1

On top of the photosensitive layer PL-01 an aqueous solution with the composition as defined in Table 2 was coated (40 *µ*m) and dried at 110°C for 2 minutes. The so-formed protective top layer OC-1 has a dry thickness or dry coating weight of 1.25 g/m².

The printing plate precursors PPP-01 to PPP-05 were obtained (see Table 3).

**Table 2: Composition of the top layer solution OC-01.**

| INGREDIENT (g) | OC-01 |
|---|---|
| Mowiol 4-88 (1) | 18,87 |
| Mowiol 8-98 (1) | 11,38 |
| Ebotec MB-SF (2) | 0,05 |
| Advantage S (3) | 0,63 |
| Lutensol A8 (4) | 0,33 |
| Water | 968,75 |
| (1) Mowiol 4-88™ and Mowiol 8-98™ are partially hydrolyzed polyvinylalcohols commercially available from KURARAY; | |
| (2) Ebotec MB-SF™ is a biocide commercially available from Troy Corporation; | |
| (3) Advantage S is commercially available from Ashland; | |
| (4) Lutensol A8™ is a surface active agent commercially available from BASF. | |

**Table 3: printing plate precursors PPP-01 to PPP-05.**

| **Printing plate precursors** | **Initiator** |
|---|---|
| PPP-01 *comparative* | HABI 1 |
| PPP-02 *inventive* | PEG-HABI 2 |
| PPP-03 *inventive* | PEG-HABI 3 |
| PPP-04 *Inventive* | PEG-HABI 1 |
| PPP-05 *comparative* | HABI-1 + MPEG350 |

### Imaging

The printing plate precursors were subsequently imaged on an Advantage DL3850 violet plate-setter available from Agfa Graphics NV, at 2400 dpi (200 lpi Agfa Balanced Screening (ABS)) (commercially available from Agfa Graphics NV and equipped with a 405 nm violet laser diode) and this at energy density of approximately 50 *µ*J/cm².

### Pre-heat

After exposure a pre-heat treatment was performed in the pre-heat unit of a VPP-68 processor - commercially available from Agfa Graphics NV - modified for single fluid processing at a speed of 1.2 m/min and a temperature measured on the backside of the printing plate precursor of 110°C.

### Processing

After the pre-heat step, the printing plate precursors were subjected to processing with VCF Gum NP (commercially available from Agfa Graphics NV) in a VPP68 processor™ (available from Agfa Graphics NV) at 24°C and a speed of 1.2 m/min, to remove the coating in the non-image areas from the support. This results in the printing plates PP-01 to PP-05.

### Printing Results and sensitivity

Printing was done on a Heidelberg GTO52 printing press equipped with a Dahlgren dampening system using K+E 800 offset ink and FS303SF 4% + IPA 10% in water as dampening solution. The lithographic performance in terms of ink acceptance and water retention obtained for the printing plates PP-01 and PP-05 (comparative plates) and PP-02 to PP-04 (inventive plates) was excellent for all the plates.

The sensitivity, defined as the energy at which 2% dot is clearly reproduced on print, was determined. The sensitivity obtained for the comparative printing plates (PP-01 and PP-05) and the inventive printing plates (PP-02 to PP-04) was excellent for all the plates.

### Evaluation of deposit formation during processing.

A specific test procedure was used to evaluate deposit formation during processing of the printing plate precursors PP-01 to PP-05. The test procedure enables to simulate potential formation of (yellow) deposits on the tank walls of a cleanout unit - which is typically designed in PVC - during processing. The procedure involves immersion of a PVC part in an exhausted gum solution (preparation see below) followed by measuring (yellow) deposit built-up.

### Test procedure

In a test room equipped with yellow safe light (Osram L36W 62) - to prevent polymerization due to actinic light -, a PVC plate (15 x 5 x 100 mm) was mounted on a string above a recipient containing exhausted gum. The PVC plate was dipped in a 25 ml exhausted gum solution (see below).

Subsequently, 0.5g of a saturated gum solution (see below) was added under stirring. The PVC part immersed in the gum dispersion was stirred overnight.

Subsequently the PVC plate is removed and the exhausted gum solution is drained. The PVC plate is rinsed using tap water and dried in open air for 30 minutes. Rinsing and drying may be carried out under white light conditions.

This procedure using the same PVC plate was carried out up to 20 cycles.

### Results

The test was carried out using the exhausted gum solutions as described in Table 4.

The results summarised in Table 4 show that
- the exhausted gum solutions including the photoinitiator of the present invention (exhausted gum solutions 2, 3, 4 and 5) do not result in (yellow) deposits on the PVC plate;
- the exhausted gum solutions including the photoinitiator of the prior art (exhausted gum solution 1), and the photoinitiator of the prior art mixed with MPEG350 (exhausted gum solution 6), induces formation of (yellow) deposits on the PVC plate.

The (yellow) deposits may be present in the form of (yellow) spots, marks, stain, smudge or the like.

**Table 4: results of the deposit test**

| | Photoinitiator* | Deposits on PVC plate** | | |
|---|---|---|---|---|
| | | After 1 exhaustion cycle | After 6 exhaustion cycles | After 20 exhaustion cycles |
| Exhausted gum solution 1 *Comparative* | HABI 1 | no | yes | yes |
| Exhausted gum solution 2 *Inventive* | PEG-HABI 1 | no | no | no |
| Exhausted gum solution 3 *Inventive* | PEG-HABI 7 | no | no | no |
| Exhausted gum solution 4 *Inventive* | PEG-HABI 18 | no | no | no |
| Exhausted gum solution 5 *Inventive* | PEG-HABI 13 | no | no | no |
| Exhausted gum solution 6 *Comparative* | Mixture of HABI 1 and MPEG350 | no | yes | yes |

| | | | | |
|---|---|---|---|---|
| *photoinitiator as defined above; **the evaluation was done visually: (yellow) deposits are present = yes no (yellow) deposits are present = no | | | | |

### Preparation of the exhausted gum solutions

The exhausted gum solution is prepared by adding typical photopolymer plate components (see Table 5) to Violet CF Gum NP (commercially available from Agfa Graphics NV):
- First, a solution of binder and monomers in MEK/Dowanol were added to 1 l VCF Gum NP commercially available from Agfa Graphics NV. Subsequently, the solvent was removed by reduced pressure distillation and the obtained solution was diluted with water to 1 l.
- Then, polyvinyl alcohol representing the overcoat was added and the solution was heated up to about 93°C and stirred for 1 hour. Afterwards, the solution was cooled to room temperature and a milky dispersion was obtained.
- Finally, to the obtained milky dispersion, a saturated solution containing a mixture of a photo-initiator and a sensitizer in phenoxy propanol was added and the exhausted gum solutions 1 and 2 were obtained.

**Table 5: Exhausted gum solutions 1 to 5**

| Ingredients (1) g | Exhausted gum solution | | | | | |
|---|---|---|---|---|---|---|
| | 1 | 2 | 3 | 4 | 5 | 6 |
| Koma 30 (2) 14%wt solution in MEK | 50 | = | = | = | = | = |
| FST 426R (2) 87.9%wt solution in Dowanol | 3.1 | = | = | = | = | = |
| Mono Z1620 (2) 29.4% solution in MEK | 34.9 | = | = | = | = | = |
| Mowiol 4-88 (3) | 25 | = | = | = | = | = |
| Fluomix (2) and Photoinitiator (2) (4) mixture in 0.5g phenoxypropanol | | | | | | |
| | | | | | | |
| Fluomix | 0,0028 | = | = | = | = | = |
| HABI 1 | 0.0405 | | | | | 0.0405 |
| PEG-HABI 1 | | 0.150 | | | | |
| PEG-HABI 7 | | | 0.0615 | | | |
| PEG-HABI 18 | | | | 0.09 | | |
| PEG-HABI 13 | | | | | 0.0615 | |
| MPEG350 (2) | | | | | | 0.981 |

| | | | | | | |
|---|---|---|---|---|---|---|
| (1) After processing 20 m² printing plate precursor having a coating layer of 1.25 g/m² and using one litre gum solution, about 25 g of coating components are present in the gum solution; (2) See Table 1 above; (3) See Table 3 above; (4) PEG-HABI 1, 7, 13 and 18: inventive photoinitiators see Examples above; | | | | | | |

## Claims

1. A negative-working lithographic printing plate precursor including (i) a support having a hydrophilic surface or which is provided with a hydrophilic layer, and (ii) a coating including a photopolymerisable layer including a photoinitiator and a polymerizable compound,
**characterised in that** the photoinitiator includes at least one structural moiety including an oligo- or poly(alkyleneglycol) represented by Formula I:
*-(L)ₚ-(OCHR^{a}CH₂)ₙ-(OCHR^{b}CH₂)ₘ-R Formula I
wherein
n represents an integer from 2 to 50;
m represents an integer from 0 to 50;
p represents 0 or 1;
L represents a linking group;
R represents a terminal group;
R^{a} and R^{b} independently represents hydrogen, an optionally substituted alkyl group and/or mixtures thereof; and
* denotes the linking to the remaining portion of the photoiniator.

2. The precursor according to claim 1 wherein R^{a} # R^{b}.

3. The precursor according to claims 1 or 2 wherein R^{a} represents hydrogen or an alkyl group and R^{b} represents hydroxymethyl.

4. The precursor according to claims 1 to 3 wherein the structural moiety includes an oligo- or poly(alkyleneglycol) represented by Formula II:
*-(L)ₚ-(OCH₂CH₂)ₙ-R Formula II
wherein
n, p, L, R and * represent the same as defined in claim 1.

5. The precursor according to claims 1 to 4 wherein the photoinitiator is a substituted bisimidazole photoinitiator.

6. The printing plate precursor according to any of the preceding claims wherein n represents an integer comprised between 3 and 20.

7. The printing plate precursor according to any of the preceding claims wherein the photoinitiator is represented by Formula III: 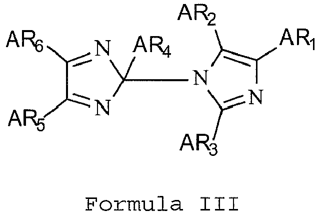 wherein
AR1 to AR6 independently represent an optionally substituted aryl, alkaryl or heteroaryl group;
with the proviso that at least one of AR1 to AR6 includes the structural moiety including an oligo- or poly(alkyleneglycol) according to Formula I.

8. The printing plate precursor according to any of the preceding claims wherein AR1 to AR6 represent an optionally substituted aryl group selected from a phenyl or naphtyl group.

9. The printing plate precursor according to any of the preceding claims wherein the photoinitiator is represented by Formula IV: 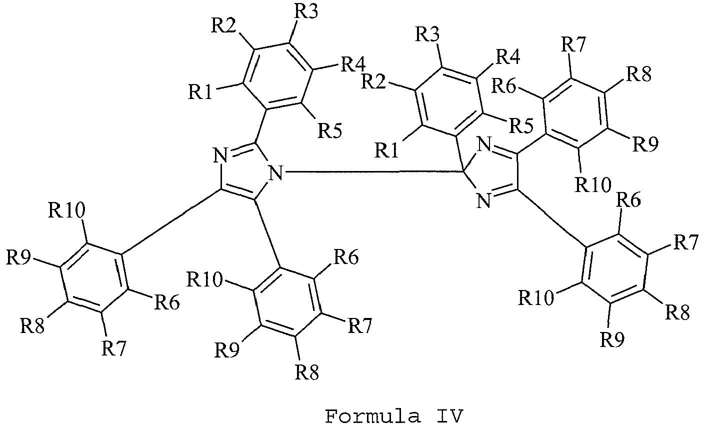
wherein R¹ to R¹⁰ are independently selected from hydrogen, an alkyl group, an alkenyl group, an alkynyl group, an aralkyl group an alkaryl group, a (hetero)aryl group, an alkoxy group, a hydroxyl group, a (hetero)aryloxy group, a halogen, a carboxyl group, an ester, an amide, a nitro group and a nitrile group;
with the proviso that at least one of R¹ to R¹⁰ represent the structural moiety including an oligo- or poly(alkyleneglycol) according to Formula I.

10. The precursor according to claim 9 wherein R¹ represents a halogen and R² to R¹⁰ represent hydrogen with the proviso that at least one of R⁶ to R¹⁰ represents the structural moiety according to Formula I as described above.

11. The precursor according to claim 10 wherein at least two of R⁶ to R¹⁰ represents the structural moiety according to Formula I including an oligo- or poly(alkyleneglycol).

12. A method of making a printing plate precursor including the steps of
- applying on a support having a hydrophilic surface or which is provided with a hydrophilic layer, a coating including a photopolymerisable layer as defined in claims 1 to 11; and
- drying the precursor.

13. A method of making a printing plate comprising the steps of:
- exposing the precursor as defined in any of the claims 1 to 11 with laser light;
- developing the exposed precursor by treating the precursor with a developing solution thereby removing the non-exposed areas of the coating from the support.

14. The method according to claim 13 wherein the developing solution is an aqueous gum solution having a pH between 4 and 10.

15. A method of making a lithographic printing plate comprising the steps of:
- exposing the precursor as defined in any of the claims 1 to 11 with laser light;
- developing the exposed precursor by mounting the exposed precursor on a plate cylinder of a lithographic printing press and rotating the plate cylinder while feeding dampening liquid and/or ink to the coating.
